# EUROPEAN PATENT APPLICATION

(11) **EP 2 597 102 A1**
(43) Date of publication of application: **29.05.2013**
(21) Application number: 11009384.6
(22) Date of filing: 25.11.2011
(51) Int. Cl.: C07K 14/54, C07K 16/24

(54) **A novel fusion protein comprising an antibody light chain and a polypeptide binding to IL-17**

(71) Applicant: Covagen AG, 8952 Zürich-Schlieren (CH)
(72) Inventor: Silacci Melkko, Michela, 8005 Zürich (CH); Toller, Isabella, 8037 Zürich (CH); Lembke, Wibke, 8049 Zürich (CH); Bänziger, Nadja, 8952 Schlieren (CH); Batey, Sarah, Westcliff-on-Sea Essex, SS0 9JT (CH); Grabulovski, Dragan, 8049 Zürich (CH); Bertschinger, Julian, 8634 Hombrechtikon (CH)
(74) Representative: Vossius & Partner

(57) **Abstract**

The present invention relates to a fusion protein comprising or consisting of the antibody light chain consisting of SEQ ID NO: 1 and a polypeptide binding to IL-17, wherein said polypeptide binding to IL-17 is located downstream of the C-terminus of said antibody light chain within said fusion protein, and wherein said polypeptide consists of the amino acid sequence of SEQ ID NO: 2, wherein (a) one, two or three amino acids within amino acids positions 10 to 19 of SEQ ID NO: 2 is/are substituted, deleted or added, (b) one, two or three amino acids within amino acids positions 29 to 36 of SEQ ID NO: 2 is/are substituted, deleted or added, and/or (c) the amino acid position 48 is substituted or deleted, provided that the polypeptide binding to IL-17 has at least 85% sequence identity to the amino acid sequence of SEQ ID NO: 2, wherein the determination of identity excludes amino acid positions 12 to 17, 31 to 34 and 48 of SEQ ID NO: 2.

SEQUENCE LISTING

<110> Covagen AG

<120> A novel fusion protein comprising an antibody light and a
      polypeptide binding to IL-17

<130> T3087 EP

<160> 127

<170> PatentIn version 3.5

<210> 1
<211> 214
<212> PRT
<213> Artificial sequence

<220>
<223> light chain of anti TNF antibody

<400> 1

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Gly Ile Arg Asn Tyr
            20                  25                  30

Leu Ala Trp Tyr Gln Gln Lys Pro Gly Lys Ala Pro Lys Leu Leu Ile
        35                  40                  45

Tyr Ala Ala Ser Thr Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Val Ala Thr Tyr Tyr Cys Gln Arg Tyr Asn Arg Ala Pro Tyr
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr

180                 185                 190

Ala Cys Glu Val Thr His Gln Gly Leu Ser Ser Pro Val Thr Lys Ser
        195                 200                 205

Phe Asn Arg Gly Glu Cys
    210

<210>  2
<211>  63
<212>  PRT
<213>  Artificial sequence

<220>
<223>  Fyn SH3 domain

<400>  2

Gly Val Thr Leu Phe Val Ala Leu Tyr Asp Tyr Glu Ala Arg Thr Glu
1               5                   10                  15

Asp Asp Leu Ser Phe His Lys Gly Glu Lys Phe Gln Ile Leu Asn Ser
            20                  25                  30

Ser Glu Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60

<210>  3
<211>  65
<212>  PRT
<213>  Artificial sequence

<220>
<223>  IL-17A binder F2

<400>  3

Gly Val Thr Leu Phe Val Ala Leu Tyr Asp Tyr Val Ala Phe Trp Pro
1               5                   10                  15

Gly Asp Leu Ser Phe His Lys Gly Glu Lys Phe Gln Ile Leu Gln Gln
            20                  25                  30

Asn Glu Leu Thr Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210>  4

<211>   65
<212>   PRT
<213>   Artificial sequence

<220>
<223>   IL-17A binder B3_1

<400>   4

Gly Val Thr Leu Phe Val Ala Leu Tyr Asp Tyr Ala Ala Phe Trp Pro
1               5                   10                  15

Gly Asp Leu Ser Phe His Lys Gly Glu Lys Phe Gln Ile Leu Lys Asn
            20                  25                  30

Arg Ala Ile Thr Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210>   5
<211>   65
<212>   PRT
<213>   Artificial sequence

<220>
<223>   IL-17A binder C2_1

<400>   5

Gly Val Thr Leu Phe Val Ala Leu Tyr Asp Tyr Leu Thr Arg Trp Lys
1               5                   10                  15

Pro Asp Leu Ser Phe His Lys Gly Glu Lys Phe Gln Ile Leu Arg Lys
            20                  25                  30

Ile Phe Asp Tyr Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210>   6
<211>   65
<212>   PRT
<213>   Artificial sequence

<220>
<223>   IL-17A binder A2

<400>   6

Gly Val Thr Leu Phe Val Ala Leu Tyr Asp Tyr Val Ala Phe Trp Pro
1               5                   10                  15

Gly Asp Leu Ser Phe His Lys Gly Glu Lys Phe Gln Ile Leu Tyr Gln
            20                  25                  30

Pro His Ala Leu Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210>  7
<211>  65
<212>  PRT
<213>  Artificial sequence

<220>
<223>  IL-17A binder A5_1

<400>  7

Gly Val Thr Leu Phe Val Ala Leu Tyr Asp Tyr Val Ala Phe Trp Pro
1               5                   10                  15

Gly Asp Leu Ser Phe His Lys Gly Glu Lys Phe Gln Ile Leu Ser Pro
            20                  25                  30

His Leu Met Phe Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210>  8
<211>  65
<212>  PRT
<213>  Artificial sequence

<220>
<223>  IL-17A binder D2_1

<400>  8

Gly Val Thr Leu Phe Val Ala Leu Tyr Asp Tyr Val Ala Phe Trp Pro
1               5                   10                  15

Gly Asp Leu Ser Phe His Lys Gly Glu Lys Phe Gln Ile Leu Asp Thr
            20                  25                  30

Leu Pro Gly Asp Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210> 9
<211> 65
<212> PRT
<213> Artificial sequence

<220>
<223> IL-17A binder E1_1

<400> 9

Gly Val Thr Leu Phe Val Ala Leu Tyr Asp Tyr Val Ala Phe Trp Pro
1               5                   10                  15

Gly Asp Leu Ser Phe His Lys Gly Glu Lys Phe Gln Ile Leu Trp Asp
            20                  25                  30

Asp Arg Pro Ala Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210> 10
<211> 65
<212> PRT
<213> Artificial sequence

<220>
<223> IL-17A binder G2_1

<400> 10

Gly Val Thr Leu Phe Val Ala Leu Tyr Asp Tyr Val Ala Phe Trp Pro
1               5                   10                  15

Gly Asp Leu Ser Phe His Lys Gly Glu Lys Phe Gln Ile Leu Thr Ser
            20                  25                  30

Asp Leu Arg Met Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210> 11
<211> 65
<212> PRT
<213> Artificial sequence

<220>
<223> IL-17A binder G4_1

<400> 11

Gly Val Thr Leu Phe Val Ala Leu Tyr Asp Tyr Val Ala Phe Trp Pro
1               5                   10                  15

Gly Asp Leu Ser Phe His Lys Gly Glu Lys Phe Gln Ile Leu Pro Asp
            20                  25                  30

Gln Leu Pro Pro Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210> 12
<211> 65
<212> PRT
<213> Artificial sequence

<220>
<223> IL-17A binder H5

<400> 12

Gly Val Thr Leu Phe Val Ala Leu Tyr Asp Tyr Val Ala Phe Trp Pro
1               5                   10                  15

Gly Asp Leu Ser Phe His Lys Gly Glu Lys Phe Gln Ile Leu Phe Ser
            20                  25                  30

Ser Tyr Thr Gln Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210> 13
<211> 65
<212> PRT

<213>  Artificial sequence

<220>
<223>  IL-17A binder B1_1

<400>  13

Gly Val Thr Leu Phe Val Ala Leu Tyr Asp Tyr Val Ala Phe Trp Pro
1               5                   10                  15

Gly Asp Leu Ser Phe His Lys Gly Glu Lys Phe Gln Ile Leu Ser Ala
            20                  25                  30

Ala Glu Pro Phe Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210>  14
<211>  65
<212>  PRT
<213>  Artificial sequence

<220>
<223>  IL-17A binder B6

<400>  14

Gly Val Thr Leu Phe Val Ala Leu Tyr Asp Tyr Val Ala Phe Trp Pro
1               5                   10                  15

Gly Asp Leu Ser Phe His Lys Gly Glu Lys Phe Gln Ile Leu Asn Met
            20                  25                  30

Pro Gln Asp Gly Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210>  15
<211>  65
<212>  PRT
<213>  Artificial sequence

<220>
<223>  IL-17A binder E6_1

<400>  15

Gly Val Thr Leu Phe Val Ala Leu Tyr Asp Tyr Val Ala Phe Trp Pro

1                   5                       10                      15

Gly Asp Leu Ser Phe His Lys Gly Glu Lys Phe Gln Ile Leu Glu Ser
            20                      25                      30

Asp Arg Asn Gly Gly Asp Trp Trp Glu Ala Arg Ser Leu Thr Thr Gly
        35                      40                      45

Glu Thr Gly Tyr Ile Pro Ser Asn Tyr Val Ala Pro Val Asp Ser Ile
    50                      55                      60

Gln
65

<210>  16
<211>  65
<212>  PRT
<213>  Artificial sequence

<220>
<223>  IL-17A binder A7_1

<400>  16

Gly Val Thr Leu Phe Val Ala Leu Tyr Asp Tyr Val Ala Phe Trp Pro
1                   5                   10                      15

Gly Asp Leu Ser Phe His Lys Gly Glu Lys Phe Gln Ile Leu Ser Asp
            20                      25                      30

Thr Asp Arg Thr Gly Asp Trp Trp Glu Ala Arg Ser Leu Thr Thr Gly
        35                      40                      45

Glu Thr Gly Tyr Ile Pro Ser Asn Tyr Val Ala Pro Val Asp Ser Ile
    50                      55                      60

Gln
65

<210>  17
<211>  65
<212>  PRT
<213>  Artificial sequence

<220>
<223>  IL-17A binder C1_1

<400>  17

Gly Val Thr Leu Phe Val Ala Leu Tyr Asp Tyr Val Ala Phe Trp Pro
1                   5                   10                      15

Gly Asp Leu Ser Phe His Lys Gly Glu Lys Phe Gln Ile Leu His Ser
            20                      25                      30

Pro Thr Gln Pro Gly Asp Trp Trp Glu Ala Arg Ser Leu Thr Thr Gly

35                  40                  45

Glu Thr Gly Tyr Ile Pro Ser Asn Tyr Val Ala Pro Val Asp Ser Ile
    50                  55                  60

Gln
65

<210>  18
<211>  65
<212>  PRT
<213>  Artificial sequence

<220>
<223>  IL-17A binder C4_1

<400>  18

Gly Val Thr Leu Phe Val Ala Leu Tyr Asp Tyr Val Ala Phe Trp Pro
1               5                   10                  15

Gly Asp Leu Ser Phe His Lys Gly Glu Lys Phe Gln Ile Leu Thr Ile
            20                  25                  30

Pro Asn Asp Ala Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210>  19
<211>  65
<212>  PRT
<213>  Artificial sequence

<220>
<223>  IL-17A binder E2_1

<400>  19

Gly Val Thr Leu Phe Val Ala Leu Tyr Asp Tyr Val Ala Phe Trp Pro
1               5                   10                  15

Gly Asp Leu Ser Phe His Lys Gly Glu Lys Phe Gln Ile Leu Pro Phe
            20                  25                  30

Gln Asp Ser Val Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln

65

<210>  20
<211>  65
<212>  PRT
<213>  Artificial sequence

<220>
<223>  IL-17A binder E5

<400>  20

Gly Val Thr Leu Phe Val Ala Leu Tyr Asp Tyr Val Ala Phe Trp Pro
1               5                   10                  15

Gly Asp Leu Ser Phe His Lys Gly Glu Lys Phe Gln Ile Leu Thr Pro
            20                  25                  30

Gln Leu Pro Val Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210>  21
<211>  65
<212>  PRT
<213>  Artificial sequence

<220>
<223>  IL-17A binder E7

<400>  21

Gly Val Thr Leu Phe Val Ala Leu Tyr Asp Tyr Val Ala Phe Trp Pro
1               5                   10                  15

Gly Asp Leu Ser Phe His Lys Gly Glu Lys Phe Gln Ile Leu Ser Ala
            20                  25                  30

His Gln Leu Asp Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210>  22
<211>  65
<212>  PRT
<213>  Artificial sequence

<220>
<223>  IL-17A binder H4

<400>  22

Gly Val Thr Leu Phe Val Ala Leu Tyr Asp Tyr Val Ala Phe Trp Pro
1               5                   10                  15

Gly Asp Leu Ser Phe His Lys Gly Glu Lys Phe Gln Ile Leu Ser His
            20                  25                  30

Asp Gln Met Phe Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210>  23
<211>  65
<212>  PRT
<213>  Artificial sequence

<220>
<223>  IL-17A binder F1_1

<400>  23

Gly Val Thr Leu Phe Val Ala Leu Tyr Asp Tyr Val Ala Phe Trp Pro
1               5                   10                  15

Gly Asp Leu Ser Phe His Lys Gly Glu Lys Phe Gln Ile Leu His Trp
            20                  25                  30

Gly Gly His Asp Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210>  24
<211>  65
<212>  PRT
<213>  Artificial sequence

<220>
<223>  IL-17A binder B3_2

<400>  24

Gly Val Thr Leu Phe Val Ala Leu Tyr Asp Tyr Val Ala Phe Trp Pro
1               5                   10                  15

Gly Asp Leu Ser Phe His Lys Gly Glu Lys Phe Gln Ile Leu His Thr
            20                  25                  30

Leu Pro Thr Asp Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210>  25
<211>  65
<212>  PRT
<213>  Artificial sequence

<220>
<223>  IL-17A binder B4_1

<400>  25

Gly Val Thr Leu Phe Val Ala Leu Tyr Asp Tyr Val Ala Phe Trp Pro
1               5                   10                  15

Gly Asp Leu Ser Phe His Lys Gly Glu Lys Phe Gln Ile Leu Ser Gly
            20                  25                  30

Pro Gln Tyr Ala Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210>  26
<211>  65
<212>  PRT
<213>  Artificial sequence

<220>
<223>  IL-17A binder C2_2

<400>  26

Gly Val Thr Leu Phe Val Ala Leu Tyr Asp Tyr Val Ala Phe Trp Pro
1               5                   10                  15

Gly Asp Leu Ser Phe His Lys Gly Glu Lys Phe Gln Ile Leu Pro His
            20                  25                  30

Lys Met Asn Asp Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210>  27
<211>  65
<212>  PRT
<213>  Artificial sequence

<220>
<223>  IL-17A binder D2_2

<400>  27

Gly Val Thr Leu Phe Val Ala Leu Tyr Asp Tyr Val Ala Phe Trp Pro
1               5                   10                  15

Gly Asp Leu Ser Phe His Lys Gly Glu Lys Phe Gln Ile Leu Arg Pro
            20                  25                  30

Thr Ile Pro Pro Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210>  28
<211>  65
<212>  PRT
<213>  Artificial sequence

<220>
<223>  IL-17A binder D3_1

<400>  28

Gly Val Thr Leu Phe Val Ala Leu Tyr Asp Tyr Val Ala Phe Trp Pro
1               5                   10                  15

Gly Asp Leu Ser Phe His Lys Gly Glu Lys Phe Gln Ile Leu Val Ser
            20                  25                  30

Asn Gln Thr Thr Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210>  29
<211>  65
<212>  PRT
<213>  Artificial sequence

<220>
<223>  IL-17A binder G4_2

<400>  29

Gly Val Thr Leu Phe Val Ala Leu Tyr Asp Tyr Val Ala Phe Trp Pro
1               5                   10                  15

Gly Asp Leu Ser Phe His Lys Gly Glu Lys Phe Gln Ile Leu Thr Val
            20                  25                  30

Pro Pro Met Gly Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210>  30
<211>  65
<212>  PRT
<213>  Artificial sequence

<220>
<223>  IL-17A binder B2_1

<400>  30

Gly Val Thr Leu Phe Val Ala Leu Tyr Asp Tyr Val Ala Phe Trp Pro
1               5                   10                  15

Gly Asp Leu Ser Phe His Lys Gly Glu Lys Phe Gln Ile Leu Ser Pro
            20                  25                  30

Gln Pro Ile Thr Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210>  31
<211>  65
<212>  PRT
<213>  Artificial sequence

<220>
<223>  IL-17A binder A1_1

<400>  31

Gly Val Thr Leu Phe Val Ala Leu Tyr Asp Tyr Val Ala Phe Trp Pro
1               5                   10                  15

Gly Asp Leu Ser Phe His Lys Gly Glu Lys Phe Gln Ile Leu Ser Ser
            20                  25                  30

Pro Lys Tyr Ala Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210>  32
<211>  65
<212>  PRT
<213>  Artificial sequence

<220>
<223>  IL-17A binder B5_1

<400>  32

Gly Val Thr Leu Phe Val Ala Leu Tyr Asp Tyr Gln Ala Phe Trp Pro
1               5                   10                  15

Gly Asp Leu Ser Phe His Lys Gly Glu Lys Phe Gln Ile Leu Thr Val
            20                  25                  30

Pro Pro Met Gly Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210>  33
<211>  65
<212>  PRT
<213>  Artificial sequence

<220>
<223>  IL-17A binder B8

<400>  33

Gly Val Thr Leu Phe Val Ala Leu Tyr Asp Tyr Ser Ala Phe Trp Pro
1               5                   10                  15

Gly Asp Leu Ser Phe His Lys Gly Glu Lys Phe Gln Ile Leu Thr Val

20                  25                  30

Pro Pro Met Gly Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210>  34
<211>  65
<212>  PRT
<213>  Artificial sequence

<220>
<223>  IL-17A binder C4_2

<400>  34

Glu Val Thr Leu Phe Val Ala Leu Tyr Asp Tyr Thr Ala Phe Trp Pro
1               5                   10                  15

Gly Asp Leu Ser Phe His Lys Gly Glu Lys Phe Gln Ile Leu Pro His
            20                  25                  30

Lys Met Asn Asp Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210>  35
<211>  65
<212>  PRT
<213>  Artificial sequence

<220>
<223>  IL-17A binder F3_1

<400>  35

Gly Val Thr Leu Phe Val Ala Leu Tyr Asp Tyr Val Ala Phe Trp Pro
1               5                   10                  15

Gly Asp Leu Ser Phe His Lys Gly Glu Lys Phe Gln Ile Leu Thr Val
            20                  25                  30

Pro Pro Met Gly Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile

50                  55                  60

Gln
65

<210>  36
<211>  65
<212>  PRT
<213>  Artificial sequence

<220>
<223>  IL-17A binder G3

<400>  36

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu His Thr
            20                  25                  30

Leu Pro Thr Asp Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210>  37
<211>  63
<212>  PRT
<213>  Artificial sequence

<220>
<223>  IL-17A binder D3_2

<400>  37

Gly Val Thr Leu Phe Val Ala Leu Tyr Asp Tyr Trp Ser Pro Trp Pro
1               5                   10                  15

Ala Asp Leu Ser Phe His Lys Gly Glu Lys Phe Gln Ile Leu Asn Ser
            20                  25                  30

Ser Glu Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60

<210>  38
<211>  63
<212>  PRT
<213>  Artificial sequence

<220>
<223>  IL-17A binder D5

<400>  38

Gly Val Thr Leu Phe Val Ala Leu Tyr Asp Tyr His Ser Pro Phe Thr
1               5                   10                  15

Phe Asp Leu Ser Phe His Lys Gly Glu Lys Phe Gln Ile Leu Asn Ser
            20                  25                  30

Ser Glu Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60

<210>  39
<211>  63
<212>  PRT
<213>  Artificial sequence

<220>
<223>  IL-17A binder D2_3

<400>  39

Gly Val Thr Leu Phe Val Ala Leu Tyr Asp Tyr Gln Ala Phe Trp Pro
1               5                   10                  15

Glu Asp Leu Ser Phe His Lys Gly Glu Lys Phe Gln Ile Leu Asn Ser
            20                  25                  30

Ser Glu Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60

<210>  40
<211>  63
<212>  PRT
<213>  Artificial sequence

<220>
<223>  IL-17A binder A3_1

<400>  40

Gly Val Thr Leu Phe Val Ala Leu Tyr Asp Tyr Gln Ala Phe Trp Pro
1               5                   10                  15

Gly Asp Leu Ser Phe His Lys Gly Glu Lys Phe Gln Ile Leu Gln Pro
            20                  25                  30

His Pro Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60

<210>  41
<211>  63
<212>  PRT
<213>  Artificial sequence

<220>
<223>  IL-17A binder B1_2

<400>  41

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Thr
            20                  25                  30

Ser Asp Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60

<210>  42
<211>  63
<212>  PRT
<213>  Artificial sequence

<220>
<223>  IL-17A binder D3_3

<400>  42

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Glu Pro
            20                  25                  30

Thr Pro Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60

<210>  43
<211>  63
<212>  PRT
<213>  Artificial sequence

<220>
<223>  IL-17A binder E2_2

<400>  43

Gly Val Thr Leu Phe Val Ala Leu Tyr Asp Tyr Thr Ala Phe Trp Pro

1               5                   10                  15

Gly Asp Leu Ser Phe His Lys Gly Glu Lys Phe Gln Ile Leu Thr Val
            20                  25                  30

Tyr Pro Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60

<210>  44
<211>  63
<212>  PRT
<213>  Artificial sequence

<220>
<223>  IL-17A binder F4

<400>  44

Gly Val Thr Leu Phe Val Ala Leu Tyr Asp Tyr Thr Ala Phe Trp Pro
1               5                   10                  15

Gly Asp Leu Ser Phe His Lys Gly Glu Lys Phe Gln Ile Leu Ser Asp
            20                  25                  30

Phe Pro Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60

<210>  45
<211>  63
<212>  PRT
<213>  Artificial sequence

<220>
<223>  IL-17A binder A6_1

<400>  45

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Thr Pro
            20                  25                  30

Ser Asp Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60

<210>  46

<211>  63
<212>  PRT
<213>  Artificial sequence

<220>
<223>  IL-17A binder B10

<400>  46

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Gln Phe
            20                  25                  30

Ser Asp Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60

<210>  47
<211>  63
<212>  PRT
<213>  Artificial sequence

<220>
<223>  IL-17A binder B12

<400>  47

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Ala Tyr
            20                  25                  30

Ser Asp Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60

<210>  48
<211>  63
<212>  PRT
<213>  Artificial sequence

<220>
<223>  IL-17A binder C5_1

<400>  48

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Tyr Ile
            20                  25                  30

Ser Asp Gly Asp Trp Trp Lys Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60

<210> 49
<211> 63
<212> PRT
<213> Artificial sequence

<220>
<223> IL-17A binder D12

<400> 49

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Gln Tyr
            20                  25                  30

Ser Asp Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60

<210> 50
<211> 63
<212> PRT
<213> Artificial sequence

<220>
<223> IL-17A binder D2_4

<400> 50

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Lys Tyr
            20                  25                  30

Ser Asp Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60

<210> 51
<211> 63
<212> PRT
<213> Artificial sequence

<220>
<223> IL-17A binder B2_2

<400>  51

Gly Val Thr Leu Phe Val Ala Leu Tyr Asp Tyr Leu Ala Phe Trp Pro
1               5                   10                  15

Gly Asp Leu Ser Phe His Lys Gly Glu Lys Phe Gln Ile Leu Arg Thr
            20                  25                  30

Ser Asp Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60

<210>  52
<211>  63
<212>  PRT
<213>  Artificial sequence

<220>
<223>  IL-17A binder D2_5

<400>  52

Gly Val Thr Leu Phe Val Ala Leu Tyr Asp Tyr Val Ala Phe Trp Pro
1               5                   10                  15

Gly Asp Leu Ser Phe His Lys Gly Glu Lys Phe Gln Ile Leu Arg Thr
            20                  25                  30

Ser Asp Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60

<210>  53
<211>  63
<212>  PRT
<213>  Artificial sequence

<220>
<223>  IL-17A binder D7_1

<400>  53

Gly Val Thr Leu Phe Val Ala Leu Tyr Asp Tyr Ile Ala Phe Trp Pro
1               5                   10                  15

Gly Asp Leu Ser Phe His Lys Gly Glu Lys Phe Gln Ile Leu Arg Thr
            20                  25                  30

Ser Asp Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln

50                  55                  60

<210>  54
<211>  63
<212>  PRT
<213>  Artificial sequence

<220>
<223>  IL-17A binder C11

<400>  54

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Thr
            20                  25                  30

Ser Asp Gly Glu Trp Trp Ile Ala Arg Ser Leu Thr Thr Gly Glu Gln
        35                  40                  45

Gly Tyr Ile Pro Ser Asn Tyr Val Ala Pro Val Asp Ser Ile Gln
    50                  55                  60

<210>  55
<211>  63
<212>  PRT
<213>  Artificial sequence

<220>
<223>  IL-17A binder A1_2

<400>  55

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Thr
            20                  25                  30

Ser Asp Gly Glu Trp Trp Ile Ala Arg Ser Leu Thr Thr Gly Glu Ser
        35                  40                  45

Gly Tyr Ile Pro Ser Asn Tyr Val Ala Pro Val Asp Ser Ile Gln
    50                  55                  60

<210>  56
<211>  63
<212>  PRT
<213>  Artificial sequence

<220>
<223>  IL-17A binder B7_1

<400>  56

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Thr
            20                  25                  30

Ser Asp Gly Glu Trp Trp Ile Ala Arg Ser Leu Thr Thr Gly Glu Arg
        35                  40                  45

Gly Tyr Ile Pro Ser Asn Tyr Val Ala Pro Val Asp Ser Ile Gln
    50                  55                  60

<210>  57
<211>  63
<212>  PRT
<213>  Artificial sequence

<220>
<223>  IL-17A binder B5_2

<400>  57

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Thr
            20                  25                  30

Ser Asp Gly Glu Trp Trp Leu Ala Arg Ser Leu Thr Thr Gly Glu Ala
        35                  40                  45

Gly Tyr Ile Pro Ser Asn Tyr Val Ala Pro Val Asp Ser Ile Gln
    50                  55                  60

<210>  58
<211>  63
<212>  PRT
<213>  Artificial sequence

<220>
<223>  IL-17A binder H11_1

<400>  58

Gly Val Thr Leu Phe Val Ala Leu Tyr Asp Tyr Lys Ala Phe Trp Pro
1               5                   10                  15

Gly Asp Val Ser Phe His Lys Gly Glu Lys Phe Gln Ile Leu Arg Thr
            20                  25                  30

Ser Asp Gly Glu Trp Trp Leu Ala Arg Ser Leu Thr Thr Gly Glu Arg
        35                  40                  45

Gly Tyr Ile Pro Ser Asn Tyr Val Ala Pro Val Asp Ser Ile Gln
    50                  55                  60

<210>  59
<211>  63
<212>  PRT

<213>  Artificial sequence

<220>
<223>  IL-17A binder E4

<400>  59

Gly Val Thr Leu Phe Val Ala Leu Tyr Asp Tyr Lys Ala Phe Trp Pro
1               5                   10                  15

Gly Asp Ile Ser Phe His Lys Gly Glu Lys Phe Gln Ile Leu Arg Thr
            20                  25                  30

Ser Asp Gly Glu Trp Trp Val Ala Arg Ser Leu Thr Thr Gly Glu Glu
        35                  40                  45

Gly Tyr Ile Pro Ser Asn Tyr Val Ala Pro Val Asp Ser Ile Gln
    50                  55                  60

<210>  60
<211>  63
<212>  PRT
<213>  Artificial sequence

<220>
<223>  IL-17A binder F3_2

<400>  60

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Thr
            20                  25                  30

Ser Asp Gly Glu Trp Trp Glu Ala Arg Ser Leu Thr Thr Gly Glu Glu
        35                  40                  45

Gly Tyr Ile Pro Ser Asn Tyr Val Ala Pro Val Asp Ser Ile Gln
    50                  55                  60

<210>  61
<211>  63
<212>  PRT
<213>  Artificial sequence

<220>
<223>  IL-17A binder A3_2

<400>  61

Gly Val Thr Leu Phe Val Ala Leu Tyr Asp Tyr Lys Ala Phe Trp Pro
1               5                   10                  15

Gly Asp Ile Ser Phe His Lys Gly Glu Lys Phe Gln Ile Leu Arg Thr
            20                  25                  30

Ser Asp Gly Glu Trp Trp Met Ala Arg Ser Leu Thr Thr Gly Glu Val

35                  40                  45

Gly Tyr Ile Pro Ser Asn Tyr Val Ala Pro Val Asp Ser Ile Gln
    50                  55                  60

<210>  62
<211>  63
<212>  PRT
<213>  Artificial sequence

<220>
<223>  IL-17A binder A5_2

<400>  62

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Thr
            20                  25                  30

Ser Asp Gly Asp Trp Trp Val Ala Arg Ser Leu Thr Thr Gly Glu Asp
        35                  40                  45

Gly Tyr Ile Pro Ser Asn Tyr Val Ala Pro Val Asp Ser Ile Gln
    50                  55                  60

<210>  63
<211>  63
<212>  PRT
<213>  Artificial sequence

<220>
<223>  IL-17A binder A8

<400>  63

Gly Val Thr Leu Phe Val Ala Leu Tyr Asp Tyr Lys Ala Phe Trp Pro
1               5                   10                  15

Gly Asp Ile Ser Phe His Lys Gly Glu Lys Phe Gln Ile Leu Arg Thr
            20                  25                  30

Ser Asp Gly Asp Trp Trp Tyr Ala Arg Ser Leu Thr Thr Gly Glu Arg
        35                  40                  45

Gly Tyr Ile Pro Ser Asn Tyr Val Ala Pro Val Asp Ser Ile Gln
    50                  55                  60

<210>  64
<211>  63
<212>  PRT
<213>  Artificial sequence

<220>
<223>  IL-17A binder H10

<400>  64

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Thr
            20                  25                  30

Ser Asp Gly Glu Trp Trp Leu Ala Arg Ser Leu Thr Thr Gly Glu Ala
        35                  40                  45

Gly Tyr Ile Pro Ser Asn Tyr Val Ala Pro Val Asp Ser Ile Gln
    50                  55                  60

<210>  65
<211>  63
<212>  PRT
<213>  Artificial sequence

<220>
<223>  IL-17A binder A9

<400>  65

Gly Val Thr Leu Phe Val Ala Leu Tyr Asp Tyr Lys Ala Phe Trp Pro
1               5                   10                  15

Gly Asp Ile Ser Phe His Lys Gly Glu Lys Phe Gln Ile Leu Arg Thr
            20                  25                  30

Ser Asp Gly Glu Trp Trp Phe Ala Arg Ser Leu Thr Thr Gly Glu Ser
        35                  40                  45

Gly Tyr Ile Pro Ser Asn Tyr Val Ala Pro Val Asp Ser Ile Gln
    50                  55                  60

<210>  66
<211>  63
<212>  PRT
<213>  Artificial sequence

<220>
<223>  IL-17A binder B2_3

<400>  66

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Thr
            20                  25                  30

Ser Asp Gly Glu Trp Trp Phe Ala Arg Ser Leu Thr Thr Gly Gly Asp
        35                  40                  45

Gly Tyr Ile Pro Ser Asn Tyr Val Ala Pro Val Asp Ser Ile Gln
    50                  55                  60

<210>  67
<211>  63
<212>  PRT
<213>  Artificial sequence

<220>
<223>  IL-17A binder A7_2

<400>  67

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Thr
            20                  25                  30

Ser Asp Gly Glu Trp Trp Phe Ala Arg Ser Leu Thr Thr Gly Glu Gly
        35                  40                  45

Gly Tyr Ile Pro Ser Asn Tyr Val Ala Pro Val Asp Ser Ile Gln
    50                  55                  60

<210>  68
<211>  63
<212>  PRT
<213>  Artificial sequence

<220>
<223>  IL-17A binder C2_3

<400>  68

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Thr
            20                  25                  30

Ser Asp Gly Glu Trp Trp Phe Ala Arg Ser Leu Thr Thr Gly Glu Asp
        35                  40                  45

Gly Tyr Ile Pro Ser Asn Tyr Val Ala Pro Val Asp Ser Ile Gln
    50                  55                  60

<210>  69
<211>  63
<212>  PRT
<213>  Artificial sequence

<220>
<223>  IL-17A binder A3_3

<400>  69

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Thr

20                      25                      30

Ser Asp Gly Glu Trp Trp Val Ala Arg Ser Leu Thr Thr Gly Glu Ala
        35                      40                      45

Gly Tyr Ile Pro Ser Asn Tyr Val Ala Pro Val Asp Ser Ile Gln
    50                      55                      60

<210>  70
<211>  63
<212>  PRT
<213>  Artificial sequence

<220>
<223>  IL-17A binder C1_2

<400>  70

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Thr
            20                  25                  30

Ser Asp Gly Glu Trp Trp Leu Ala Arg Ser Leu Thr Thr Gly Glu Ala
        35                  40                  45

Gly Tyr Ile Pro Ser Asn Tyr Val Ala Pro Val Asp Ser Ile Gln
    50                  55                  60

<210>  71
<211>  63
<212>  PRT
<213>  Artificial sequence

<220>
<223>  IL-17A binder E9

<400>  71

Gly Val Thr Leu Phe Val Ala Leu Tyr Asp Tyr Lys Ala Phe Trp Pro
1               5                   10                  15

Gly Asp Ile Ser Phe His Lys Gly Glu Lys Phe Gln Ile Leu Arg Thr
            20                  25                  30

Ser Asp Gly Glu Trp Trp Phe Ala Arg Ser Leu Thr Thr Gly Glu Ala
        35                  40                  45

Gly Tyr Ile Pro Ser Asn Tyr Val Ala Pro Val Asp Ser Ile Gln
    50                  55                  60

<210>  72
<211>  63
<212>  PRT
<213>  Artificial sequence

<220>
<223>  IL-17A binder D1

<400>  72

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Thr
            20                  25                  30

Ser Asp Gly Glu Trp Trp Ser Ala Arg Ser Leu Thr Thr Gly Glu Glu
        35                  40                  45

Gly Tyr Ile Pro Ser Asn Tyr Val Ala Pro Val Asp Ser Ile Gln
    50                  55                  60

<210>  73
<211>  63
<212>  PRT
<213>  Artificial sequence

<220>
<223>  IL-17A binder B3_3

<400>  73

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Thr
            20                  25                  30

Ser Asp Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60

<210>  74
<211>  63
<212>  PRT
<213>  Artificial sequence

<220>
<223>  IL-17A binder A12

<400>  74

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Thr Leu
            20                  25                  30

Ser Asp Gly Glu Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60

<210>  75
<211>  63
<212>  PRT
<213>  Artificial sequence

<220>
<223>  IL-17A binder H3

<400>  75

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Leu Glu
            20                  25                  30

Ser Asp Gly Ser Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60

<210>  76
<211>  63
<212>  PRT
<213>  Artificial sequence

<220>
<223>  IL-17A binder H9

<400>  76

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Gln Val
            20                  25                  30

Ser Asp Gly Glu Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60

<210>  77
<211>  63
<212>  PRT
<213>  Artificial sequence

<220>
<223>  IL-17A binder F1_2

<400>  77

Gly Val Thr Leu Phe Val Ala Leu Tyr Asp Tyr Lys Ala Phe Trp Pro

1               5                   10                  15

Gly Asp Leu Ser Phe His Lys Gly Glu Lys Phe Gln Ile Leu Arg Thr
            20                  25                  30

Ser Asp Gly Glu Trp Trp Glu Ala Arg Ser Leu Thr Thr Gly Glu Lys
        35                  40                  45

Gly Tyr Ile Pro Ser Asn Tyr Val Ala Pro Val Asp Ser Ile Gln
    50                  55                  60

<210>  78
<211>  63
<212>  PRT
<213>  Artificial sequence

<220>
<223>  IL-17A binder F10

<400>  78

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Thr
            20                  25                  30

Ser Asp Gly Glu Trp Trp Glu Ala Arg Ser Leu Thr Thr Gly Glu Glu
        35                  40                  45

Gly Tyr Ile Pro Ser Asn Tyr Val Ala Pro Val Asp Ser Ile Gln
    50                  55                  60

<210>  79
<211>  63
<212>  PRT
<213>  Artificial sequence

<220>
<223>  IL-17A binder C5_2

<400>  79

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Thr
            20                  25                  30

Ser Asp Gly Glu Trp Trp Glu Ala Arg Ser Leu Thr Thr Gly Glu Tyr
        35                  40                  45

Gly Tyr Ile Pro Ser Asn Tyr Val Ala Pro Val Asp Ser Ile Gln
    50                  55                  60

<210>  80

<211>  63
<212>  PRT
<213>  Artificial sequence

<220>
<223>  IL-17A binder D7_2

<400>  80

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Thr
            20                  25                  30

Ser Asp Gly Asp Trp Trp Glu Ala Arg Ser Leu Thr Thr Gly Glu Tyr
        35                  40                  45

Gly Tyr Ile Pro Ser Asn Tyr Val Ala Pro Val Asp Ser Ile Gln
    50                  55                  60

<210>  81
<211>  63
<212>  PRT
<213>  Artificial sequence

<220>
<223>  IL-17A binder C2_4

<400>  81

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Thr
            20                  25                  30

Ser Asp Gly Asp Trp Trp Glu Ala Arg Ser Leu Thr Thr Gly Glu Ser
        35                  40                  45

Gly Tyr Ile Pro Ser Asn Tyr Val Ala Pro Val Asp Ser Ile Gln
    50                  55                  60

<210>  82
<211>  63
<212>  PRT
<213>  Artificial sequence

<220>
<223>  IL-17A binder E3_1

<400>  82

Gly Val Thr Leu Phe Val Ala Leu Tyr Asp Tyr Lys Ala Phe Trp Pro
1               5                   10                  15

Gly Asp Ile Ser Phe His Lys Gly Glu Lys Phe Gln Ile Leu Arg Thr
            20                  25                  30

Ser Asp Gly Ala Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60

<210>  83
<211>  63
<212>  PRT
<213>  Artificial sequence

<220>
<223>  IL-17A binder A11

<400>  83

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Thr
            20                  25                  30

Ser Asp Gly Glu Trp Trp Leu Ala Arg Ser Leu Thr Thr Gly Glu Glu
        35                  40                  45

Gly Tyr Ile Pro Ser Asn Tyr Val Ala Pro Val Asp Ser Ile Gln
    50                  55                  60

<210>  84
<211>  63
<212>  PRT
<213>  Artificial sequence

<220>
<223>  IL-17A binder B4_2

<400>  84

Gly Val Thr Leu Phe Val Ala Leu Tyr Asp Tyr Lys Ala Phe Trp Pro
1               5                   10                  15

Gly Asp Val Ser Phe His Lys Gly Glu Lys Phe Gln Ile Leu Arg Thr
            20                  25                  30

Ser Asp Gly Glu Trp Trp Leu Ala Arg Ser Leu Thr Thr Gly Glu Tyr
        35                  40                  45

Gly Tyr Ile Pro Ser Asn Tyr Val Ala Pro Val Asp Ser Ile Gln
    50                  55                  60

<210>  85
<211>  63
<212>  PRT
<213>  Artificial sequence

<220>
<223>  IL-17A binder D10

<400>  85

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Thr
            20                  25                  30

Ser Asp Gly Glu Trp Trp Ser Ala Arg Ser Leu Thr Thr Gly Glu Val
        35                  40                  45

Gly Tyr Ile Pro Ser Asn Tyr Val Ala Pro Val Asp Ser Ile Gln
    50                  55                  60

<210>  86
<211>  63
<212>  PRT
<213>  Artificial sequence

<220>
<223>  IL-17A binder E6_2

<400>  86

Gly Val Thr Leu Phe Val Ala Leu Tyr Asp Tyr Lys Ala Phe Trp Pro
1               5                   10                  15

Gly Asp Ile Ser Phe His Lys Gly Glu Lys Phe Gln Ile Leu Arg Thr
            20                  25                  30

Ser Asp Gly Leu Trp Trp Phe Ala Arg Ser Leu Thr Thr Gly Glu Asp
        35                  40                  45

Gly Tyr Ile Pro Ser Asn Tyr Val Ala Pro Val Asp Ser Ile Gln
    50                  55                  60

<210>  87
<211>  63
<212>  PRT
<213>  Artificial sequence

<220>
<223>  IL-17A binder H11_2

<400>  87

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Thr
            20                  25                  30

Ser Asp Gly Glu Trp Trp Glu Ala Arg Ser Leu Thr Thr Gly Glu Glu
        35                  40                  45

Gly Tyr Ile Pro Ser Asn Tyr Val Ala Pro Val Asp Ser Ile Gln

50                  55                  60

<210>  88
<211>  63
<212>  PRT
<213>  Artificial sequence

<220>
<223>  IL-17A binder A5_3

<400>  88

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Thr
            20                  25                  30

Ser Asp Gly Asn Trp Trp Glu Ala Arg Ser Leu Thr Thr Gly Glu Ala
        35                  40                  45

Gly Tyr Ile Pro Ser Asn Tyr Val Ala Pro Val Asp Ser Ile Gln
    50                  55                  60

<210>  89
<211>  63
<212>  PRT
<213>  Artificial sequence

<220>
<223>  IL-17A binder F12

<400>  89

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Thr
            20                  25                  30

Ser Asp Gly Glu Trp Trp Asp Ala Arg Ser Leu Thr Thr Gly Glu Arg
        35                  40                  45

Gly Tyr Ile Pro Ser Asn Tyr Val Ala Pro Val Asp Ser Ile Gln
    50                  55                  60

<210>  90
<211>  63
<212>  PRT
<213>  Artificial sequence

<220>
<223>  IL-17A binder B7_2

<400>  90

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Thr
            20                  25                  30

Ser Asp Gly Asp Trp Trp Val Ala Arg Ser Leu Thr Thr Gly Glu Asp
        35                  40                  45

Gly Tyr Ile Pro Ser Asn Tyr Val Ala Pro Val Asp Ser Ile Gln
    50                  55                  60

<210>  91
<211>  63
<212>  PRT
<213>  Artificial sequence

<220>
<223>  IL-17A binder G2_2

<400>  91

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Gly
            20                  25                  30

Ser Asp Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60

<210>  92
<211>  63
<212>  PRT
<213>  Artificial sequence

<220>
<223>  IL-17A binder C5_3

<400>  92

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Thr
            20                  25                  30

Ser Asp Gly Tyr Trp Trp Glu Ala Arg Ser Leu Thr Thr Gly Glu Asn
        35                  40                  45

Gly Tyr Ile Pro Ser Asn Tyr Val Ala Pro Val Asp Ser Ile Gln
    50                  55                  60

<210>  93
<211>  63
<212>  PRT

<213>  Artificial sequence

<220>
<223>  IL-17A binder G11

<400>  93

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Thr
            20                  25                  30

Ser Asp Gly Asp Trp Trp Thr Ala Arg Ser Leu Thr Thr Gly Glu Glu
        35                  40                  45

Gly Tyr Ile Pro Ser Asn Tyr Val Ala Pro Val Asp Ser Ile Gln
    50                  55                  60

<210>  94
<211>  63
<212>  PRT
<213>  Artificial sequence

<220>
<223>  IL-17A binder F5

<400>  94

Gly Val Thr Leu Phe Val Ala Leu Tyr Asp Tyr Lys Ala Phe Trp Pro
1               5                   10                  15

Gly Asp Met Ser Phe His Lys Gly Glu Lys Phe Gln Ile Leu Arg Thr
            20                  25                  30

Ser Asp Gly Ile Trp Trp Ile Ala Arg Ser Leu Thr Thr Gly Glu Glu
        35                  40                  45

Gly Tyr Ile Pro Ser Asn Tyr Val Ala Pro Val Asp Ser Ile Gln
    50                  55                  60

<210>  95
<211>  63
<212>  PRT
<213>  Artificial sequence

<220>
<223>  IL-17A binder D3_4

<400>  95

Gly Val Thr Leu Phe Val Ala Leu Tyr Asp Tyr Lys Ala Phe Trp Pro
1               5                   10                  15

Gly Asp Ile Ser Phe His Lys Gly Glu Lys Phe Gln Ile Leu Arg Thr
            20                  25                  30

Ser Asp Gly Gln Trp Trp Ile Ala Arg Ser Leu Thr Thr Gly Glu Glu

35                  40                  45

Gly Tyr Ile Pro Ser Asn Tyr Val Ala Pro Val Asp Ser Ile Gln
    50                  55                  60

<210>  96
<211>  63
<212>  PRT
<213>  Artificial sequence

<220>
<223>  IL-17A binder F9

<400>  96

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Thr
            20                  25                  30

Ser Asp Gly Ala Trp Trp Met Ala Arg Ser Leu Thr Thr Gly Glu Asn
        35                  40                  45

Gly Tyr Ile Pro Ser Asn Tyr Val Ala Pro Val Asp Ser Ile Gln
    50                  55                  60

<210>  97
<211>  63
<212>  PRT
<213>  Artificial sequence

<220>
<223>  IL-17A binder F1_3

<400>  97

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Thr
            20                  25                  30

Ser Asp Gly Glu Trp Trp Tyr Ala Arg Ser Leu Thr Thr Gly Glu Lys
        35                  40                  45

Gly Tyr Ile Pro Ser Asn Tyr Val Ala Pro Val Asp Ser Ile Gln
    50                  55                  60

<210>  98
<211>  63
<212>  PRT
<213>  Artificial sequence

<220>
<223>  IL-17A binder A4

<400>  98

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Glu Gly
            20                  25                  30

Ser Trp Gly Glu Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60

<210>  99
<211>  63
<212>  PRT
<213>  Artificial sequence

<220>
<223>  IL-17A binder G5

<400>  99

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Ala Ile Thr Arg Thr
            20                  25                  30

Ser Asp Gly Asp Trp Trp Glu Ala Val Ser Leu Thr Thr Gly Glu Thr
        35                  40                  45

Gly Tyr Ile Pro Ser Asn Tyr Val Ala Pro Val Asp Ser Ile Gln
    50                  55                  60

<210>  100
<211>  63
<212>  PRT
<213>  Artificial sequence

<220>
<223>  IL-17A binder A6_2

<400>  100

Gly Val Thr Leu Phe Val Ala Leu Tyr Asp Tyr Lys Ala Phe Trp Pro
1               5                   10                  15

Gly Asp Ile Ser Phe His Lys Gly Glu Lys Phe Gln Ile Leu Arg Thr
            20                  25                  30

Ser Asp Gly Glu Trp Trp Val Ala Lys Ser Leu Thr Thr Gly Glu Glu
        35                  40                  45

Gly Tyr Ile Pro Ser Asn Tyr Val Ala Pro Val Asp Ser Ile Gln
    50                  55                  60

<210>  101
<211>  63
<212>  PRT
<213>  Artificial sequence

<220>
<223>  IL-17A binder D2_6

<400>  101

Gly Val Thr Leu Phe Val Ala Leu Tyr Asp Tyr Lys Ala Phe Trp Pro
1               5                   10                  15

Gly Asp Ile Ser Phe His Lys Gly Glu Lys Phe Thr Ile Leu Arg Thr
            20                  25                  30

Ser Asp Gly Glu Trp Trp Val Ala Arg Ser Leu Thr Thr Gly Glu Glu
        35                  40                  45

Gly Tyr Ile Pro Ser Asn Tyr Val Ala Pro Val Asp Ser Ile Gln
    50                  55                  60

<210>  102
<211>  63
<212>  PRT
<213>  Artificial sequence

<220>
<223>  IL-17A binder G4_3

<400>  102

Gly Val Thr Leu Phe Val Ala Leu Tyr Asp Tyr Lys Ala Phe Trp Pro
1               5                   10                  15

Gly Asp Ile Ser Phe His Lys Gly Glu Lys Phe Leu Ile Thr Arg Thr
            20                  25                  30

Ser Asp Gly Glu Trp Trp Val Ala Val Ser Leu Thr Thr Gly Glu Glu
        35                  40                  45

Gly Tyr Ile Pro Ser Asn Tyr Val Ala Pro Val Asp Ser Ile Gln
    50                  55                  60

<210>  103
<211>  63
<212>  PRT
<213>  Artificial sequence

<220>
<223>  IL-17A binder E3_2

<400>  103

Gly Val Thr Leu Phe Val Ala Leu Tyr Asp Tyr Lys Ala Phe Trp Pro
1               5                   10                  15

Gly Asp Ile Ser Phe His Lys Gly Glu Lys Phe Glu Ile Val Arg Thr

20                  25                  30

Ser Asp Gly Glu Trp Trp Val Ala Phe Ser Leu Thr Thr Gly Glu Glu
        35                  40                  45

Gly Tyr Ile Pro Ser Asn Tyr Val Ala Pro Val Asp Ser Ile Gln
    50                  55                  60

<210>  104
<211>  63
<212>  PRT
<213>  Artificial sequence

<220>
<223>  IL-17A binder B4_3

<400>  104

Gly Val Thr Leu Phe Val Ala Leu Tyr Asp Tyr Lys Ala Phe Trp Pro
1               5                   10                  15

Gly Asp Ile Ser Phe His Lys Gly Glu Lys Phe Leu Ile Leu Arg Thr
            20                  25                  30

Ser Asp Gly Glu Trp Trp Val Ala Gln Ser Leu Thr Thr Gly Glu Glu
        35                  40                  45

Gly Tyr Ile Pro Ser Asn Tyr Val Ala Pro Val Asp Ser Ile Gln
    50                  55                  60

<210>  105
<211>  63
<212>  PRT
<213>  Artificial sequence

<220>
<223>  IL-17A binder C4_3

<400>  105

Gly Val Thr Leu Phe Val Ala Leu Tyr Asp Tyr Lys Ala Phe Trp Pro
1               5                   10                  15

Gly Asp Ile Ser Phe His Lys Gly Glu Lys Phe His Ile Leu Arg Thr
            20                  25                  30

Ser Asp Gly Glu Trp Trp Val Ala Arg Ser Leu Thr Thr Gly Glu Glu
        35                  40                  45

Gly Tyr Ile Pro Ser Asn Tyr Val Ala Pro Val Asp Ser Ile Gln
    50                  55                  60

<210>  106
<211>  63
<212>  PRT
<213>  Artificial sequence

<220>
<223>  IL-17A binder C9

<400>  106

Gly Val Thr Leu Phe Val Ala Leu Tyr Asp Tyr Lys Ala Phe Trp Pro
1               5                   10                  15

Gly Asp Ile Ser Phe His Lys Gly Glu Lys Phe Phe Ile Leu Arg Thr
            20                  25                  30

Ser Asp Gly Glu Trp Trp Val Ala Gln Ser Leu Thr Thr Gly Glu Glu
        35                  40                  45

Gly Tyr Ile Pro Ser Asn Tyr Val Ala Pro Val Asp Ser Ile Gln
    50                  55                  60

<210>  107
<211>  63
<212>  PRT
<213>  Artificial sequence

<220>
<223>  IL-17A binder E1_2

<400>  107

Gly Val Thr Leu Phe Val Ala Leu Tyr Asp Tyr Lys Ala Phe Trp Pro
1               5                   10                  15

Gly Asp Ile Ser Phe His Lys Gly Glu Lys Phe Gln Ile Leu Arg Thr
            20                  25                  30

Ile Asp Gly Glu Trp Trp Val Ala Arg Ser Leu Thr Thr Gly Glu Glu
        35                  40                  45

Gly Tyr Ile Pro Ser Asn Tyr Val Ala Pro Val Asp Ser Ile Gln
    50                  55                  60

<210>  108
<211>  63
<212>  PRT
<213>  Artificial sequence

<220>
<223>  IL-17A binder H2

<400>  108

Gly Val Thr Leu Phe Val Ala Leu Tyr Asp Tyr Lys Ala Phe Trp Pro
1               5                   10                  15

Gly Asp Ile Ser Phe His Lys Gly Glu Lys Phe Arg Ile Lys Arg Thr
            20                  25                  30

Ser Asp Gly Glu Trp Trp Val Ala Val Ser Leu Thr Thr Gly Glu Glu
        35                  40                  45

Gly Tyr Ile Pro Ser Asn Tyr Val Ala Pro Val Asp Ser Ile Gln
    50                  55                  60

<210>  109
<211>  63
<212>  PRT
<213>  Artificial sequence

<220>
<223>  IL-17A binder 2C1

<400>  109

Gly Val Thr Leu Phe Val Ala Leu Tyr Asp Tyr Lys Ala Phe Trp Pro
1               5                   10                  15

Gly Asp Ile Ser Phe His Lys Gly Glu Lys Phe Gln Ile Leu Arg Thr
            20                  25                  30

Ser Asp Gly Glu Trp Trp Ile Ala Arg Ser Leu Thr Thr Gly Glu Glu
        35                  40                  45

Gly Tyr Ile Pro Ser Asn Tyr Val Ala Pro Val Asp Ser Ile Gln
    50                  55                  60

<210>  110
<211>  63
<212>  PRT
<213>  Artificial sequence

<220>
<223>  IL-17A binder 1E2

<400>  110

Gly Val Thr Leu Phe Val Ala Leu Tyr Asp Tyr Arg Ala Phe Trp Pro
1               5                   10                  15

Gly Asp Ile Ser Phe His Lys Gly Glu Lys Phe Gln Ile Leu Arg Thr
            20                  25                  30

Ser Asp Gly Glu Trp Trp Val Ala Arg Ser Leu Thr Thr Gly Glu Glu
        35                  40                  45

Gly Tyr Ile Pro Ser Asn Tyr Val Ala Pro Val Asp Ser Ile Gln
    50                  55                  60

<210>  111
<211>  63
<212>  PRT
<213>  Artificial sequence

<220>
<223>  IL-17A binder 1E9

<400>  111

Gly Val Thr Leu Phe Val Ala Leu Tyr Asp Tyr Lys Ala Ile Trp Pro

1                   5                   10                  15

Thr Asp Ile Ser Phe His Lys Gly Glu Lys Phe Gln Ile Leu Arg Thr
            20                  25                  30

Ser Asp Gly Glu Trp Trp Val Ala Arg Ser Leu Thr Thr Gly Glu Glu
        35                  40                  45

Gly Tyr Ile Pro Ser Asn Tyr Val Ala Pro Val Asp Ser Ile Gln
    50                  55                  60

<210>  112
<211>  63
<212>  PRT
<213>  Artificial sequence

<220>
<223>  IL-17A binder 1F3

<400>  112

Gly Val Thr Leu Phe Val Ala Leu Tyr Asp Tyr Lys Ala Phe Gly Pro
1               5                   10                  15

Gly Asp Ile Ser Phe His Lys Gly Glu Lys Phe Gln Ile Leu Arg Thr
            20                  25                  30

Ser Asp Gly Glu Trp Trp Val Ala Arg Ser Leu Thr Thr Gly Glu Glu
        35                  40                  45

Gly Tyr Ile Pro Ser Asn Tyr Val Ala Pro Val Asp Ser Ile Gln
    50                  55                  60

<210>  113
<211>  63
<212>  PRT
<213>  Artificial sequence

<220>
<223>  IL-17A binder 2A6

<400>  113

Gly Val Thr Leu Phe Val Ala Leu Tyr Asp Tyr Lys Ala Phe Trp Pro
1               5                   10                  15

Gly Asp Ile Ser Phe His Lys Gly Glu Lys Phe Gln Ile Leu His Thr
            20                  25                  30

Ser Asp Gly Glu Trp Trp Val Ala Arg Ser Leu Thr Thr Gly Glu Glu
        35                  40                  45

Gly Tyr Ile Pro Ser Asn Tyr Val Ala Pro Val Asp Ser Ile Gln
    50                  55                  60

<210>  114

<211>  63
<212>  PRT
<213>  Artificial sequence

<220>
<223>  IL-17A binder 34D3

<400>  114

Gly Val Thr Leu Phe Val Ala Leu Tyr Asp Tyr Lys Ala Phe Trp Pro
1               5                   10                  15

Gly Asp Ile Ser Phe His Lys Gly Glu Lys Phe Gln Ile Leu Arg Thr
            20                  25                  30

Arg Asp Gly Glu Trp Trp Val Ala Arg Ser Leu Thr Thr Gly Glu Glu
        35                  40                  45

Gly Tyr Ile Pro Ser Asn Tyr Val Ala Pro Val Asp Ser Ile Gln
    50                  55                  60

<210>  115
<211>  63
<212>  PRT
<213>  Artificial sequence

<220>
<223>  IL-17A binder COMB3

<400>  115

Gly Val Thr Leu Phe Val Ala Leu Tyr Asp Tyr Lys Ala Phe Trp Pro
1               5                   10                  15

Gly Asp Leu Ser Phe His Lys Gly Glu Lys Phe Gln Ile Leu Arg Thr
            20                  25                  30

Ser Asp Gly Glu Trp Trp Ile Ala Arg Ser Leu Thr Thr Gly Glu Glu
        35                  40                  45

Gly Tyr Ile Pro Ser Asn Tyr Val Ala Pro Val Asp Ser Ile Gln
    50                  55                  60

<210>  116
<211>  63
<212>  PRT
<213>  Artificial sequence

<220>
<223>  IL-17A binder COMB4

<400>  116

Gly Val Thr Leu Phe Val Ala Leu Tyr Asp Tyr Lys Ala Phe Trp Pro
1               5                   10                  15

Gly Asp Ile Ser Phe His Lys Gly Glu Lys Phe Gln Ile Leu Arg Thr
            20                  25                  30

Ser Asp Gly Glu Trp Trp Ile Ala Arg Ser Leu Thr Thr Gly Glu Ser
        35                  40                  45

Gly Tyr Ile Pro Ser Asn Tyr Val Ala Pro Val Asp Ser Ile Gln
    50                  55                  60

<210>  117
<211>  63
<212>  PRT
<213>  Artificial sequence

<220>
<223>  IL-17A binder COMB5

<400>  117

Gly Val Thr Leu Phe Val Ala Leu Tyr Asp Tyr Lys Ala Phe Trp Pro
1               5                   10                  15

Gly Asp Ile Ser Phe His Lys Gly Glu Lys Phe Gln Ile Leu His Thr
            20                  25                  30

Ser Asp Gly Glu Trp Trp Ile Ala Arg Ser Leu Thr Thr Gly Glu Glu
        35                  40                  45

Gly Tyr Ile Pro Ser Asn Tyr Val Ala Pro Val Asp Ser Ile Gln
    50                  55                  60

<210>  118
<211>  63
<212>  PRT
<213>  Artificial sequence

<220>
<223>  IL-17A binder COMB6

<400>  118

Gly Val Thr Leu Phe Val Ala Leu Tyr Asp Tyr Lys Ala Phe Trp Pro
1               5                   10                  15

Gly Asp Ile Ser Phe His Lys Gly Glu Lys Phe Gln Ile Leu Arg Thr
            20                  25                  30

Ser Asp Gly Glu Trp Trp Ile Ala Lys Ser Leu Thr Thr Gly Glu Glu
        35                  40                  45

Gly Tyr Ile Pro Ser Asn Tyr Val Ala Pro Val Asp Ser Ile Gln
    50                  55                  60

<210>  119
<211>  451
<212>  PRT
<213>  Artificial sequence

<220>
<223>  heavy chain of anti TNF antibody

<400>  119

Glu Val Gln Leu Val Glu Ser Gly Gly Gly Leu Val Gln Pro Gly Arg
1               5                   10                  15

Ser Leu Arg Leu Ser Cys Ala Ala Ser Gly Phe Thr Phe Asp Asp Tyr
            20                  25                  30

Ala Met His Trp Val Arg Gln Ala Pro Gly Lys Gly Leu Glu Trp Val
        35                  40                  45

Ser Ala Ile Thr Trp Asn Ser Gly His Ile Asp Tyr Ala Asp Ser Val
    50                  55                  60

Glu Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Lys Val Ser Tyr Leu Ser Thr Ala Ser Ser Leu Asp Tyr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His

260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Glu Glu Met Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Lys
    450

<210>  120
<211>  15
<212>  PRT
<213>  Artificial sequence

<220>
<223>  linker 1

<400>  120

Gly Gly Gly Gly Ser Gly Gly Gly Gly Ser Gly Gly Gly Gly Ser
1               5                   10                  15

<210>  121
<211>  10
<212>  PRT

<213>  Artificial sequence

<220>
<223>  linker 2

<400>  121

Gly Gly Gly Gly Ser Gly Gly Gly Gly Ser
1               5                   10


<210>  122
<211>  5
<212>  PRT
<213>  Artificial sequence

<220>
<223>  linker 3

<400>  122

Gly Gly Gly Gly Ser
1               5


<210>  123
<211>  19
<212>  PRT
<213>  Artificial sequence

<220>
<223>  leader sequence

<400>  123

Met Asn Phe Gly Leu Arg Leu Ile Phe Leu Val Leu Thr Leu Lys Gly
1               5                   10                  15


Val Gln Cys



<210>  124
<211>  292
<212>  PRT
<213>  Artificial sequence

<220>
<223>  fusion protein (light chain of anti TNF antibody linked to
        Fynomer 2C1)

<400>  124

Asp Ile Gln Met Thr Gln Ser Pro Ser Ser Leu Ser Ala Ser Val Gly
1               5                   10                  15


Asp Arg Val Thr Ile Thr Cys Arg Ala Ser Gln Gly Ile Arg Asn Tyr
            20                  25                  30


Leu Ala Trp Tyr Gln Gln Lys Pro Gly Lys Ala Pro Lys Leu Leu Ile
        35                  40                  45


Tyr Ala Ala Ser Thr Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Val Ala Thr Tyr Tyr Cys Gln Arg Tyr Asn Arg Ala Pro Tyr
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys Gly Gly Gly Gly Ser Gly Gly Gly Gly Ser
    210                 215                 220

Gly Gly Gly Gly Ser Gly Val Thr Leu Phe Val Ala Leu Tyr Asp Tyr
225                 230                 235                 240

Lys Ala Phe Trp Pro Gly Asp Ile Ser Phe His Lys Gly Glu Lys Phe
                245                 250                 255

Gln Ile Leu Arg Thr Ser Asp Gly Glu Trp Trp Ile Ala Arg Ser Leu
            260                 265                 270

Thr Thr Gly Glu Glu Gly Tyr Ile Pro Ser Asn Tyr Val Ala Pro Val
        275                 280                 285

Asp Ser Ile Gln
    290

<210>  125
<211>  529
<212>  PRT
<213>  Artificial sequence

<220>

<223>  heavy chain of anti TNF antibody linked to Fynomer 2C1

<400>  125

Glu Val Gln Leu Val Glu Ser Gly Gly Gly Leu Val Gln Pro Gly Arg
1               5                   10                  15

Ser Leu Arg Leu Ser Cys Ala Ala Ser Gly Phe Thr Phe Asp Asp Tyr
            20                  25                  30

Ala Met His Trp Val Arg Gln Ala Pro Gly Lys Gly Leu Glu Trp Val
        35                  40                  45

Ser Ala Ile Thr Trp Asn Ser Gly His Ile Asp Tyr Ala Asp Ser Val
    50                  55                  60

Glu Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Lys Val Ser Tyr Leu Ser Thr Ala Ser Ser Leu Asp Tyr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Glu Glu Met Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Lys Gly Gly Gly Gly Ser Gly Gly Gly Gly Ser Gly Gly Gly
    450                 455                 460

Gly Ser Gly Val Thr Leu Phe Val Ala Leu Tyr Asp Tyr Lys Ala Phe
465                 470                 475                 480

Trp Pro Gly Asp Ile Ser Phe His Lys Gly Glu Lys Phe Gln Ile Leu
                485                 490                 495

Arg Thr Ser Asp Gly Glu Trp Trp Ile Ala Arg Ser Leu Thr Thr Gly
            500                 505                 510

Glu Glu Gly Tyr Ile Pro Ser Asn Tyr Val Ala Pro Val Asp Ser Ile
        515                 520                 525

Gln

<210> 126
<211> 529
<212> PRT
<213> Artificial sequence

<220>
<223> Fynomer 2C1 linked to heavy chain of anti TNF antibody

<400> 126

Gly Val Thr Leu Phe Val Ala Leu Tyr Asp Tyr Lys Ala Phe Trp Pro
1               5                   10                  15

Gly Asp Ile Ser Phe His Lys Gly Glu Lys Phe Gln Ile Leu Arg Thr
            20                  25                  30

Ser Asp Gly Glu Trp Trp Ile Ala Arg Ser Leu Thr Thr Gly Glu Glu
        35                  40                  45

Gly Tyr Ile Pro Ser Asn Tyr Val Ala Pro Val Asp Ser Ile Gln Gly
    50                  55                  60

Gly Gly Gly Ser Gly Gly Gly Gly Ser Gly Gly Gly Gly Ser Glu Val
65                  70                  75                  80

Gln Leu Val Glu Ser Gly Gly Gly Leu Val Gln Pro Gly Arg Ser Leu
                85                  90                  95

Arg Leu Ser Cys Ala Ala Ser Gly Phe Thr Phe Asp Asp Tyr Ala Met
            100                 105                 110

His Trp Val Arg Gln Ala Pro Gly Lys Gly Leu Glu Trp Val Ser Ala
        115                 120                 125

Ile Thr Trp Asn Ser Gly His Ile Asp Tyr Ala Asp Ser Val Glu Gly
    130                 135                 140

Arg Phe Thr Ile Ser Arg Asp Asn Ala Lys Asn Ser Leu Tyr Leu Gln
145                 150                 155                 160

Met Asn Ser Leu Arg Ala Glu Asp Thr Ala Val Tyr Tyr Cys Ala Lys
                165                 170                 175

Val Ser Tyr Leu Ser Thr Ala Ser Ser Leu Asp Tyr Trp Gly Gln Gly
            180                 185                 190

Thr Leu Val Thr Val Ser Ser Ala Ser Thr Lys Gly Pro Ser Val Phe
        195                 200                 205

Pro Leu Ala Pro Ser Ser Lys Ser Thr Ser Gly Gly Thr Ala Ala Leu
    210                 215                 220

Gly Cys Leu Val Lys Asp Tyr Phe Pro Glu Pro Val Thr Val Ser Trp
225                 230                 235                 240

Asn Ser Gly Ala Leu Thr Ser Gly Val His Thr Phe Pro Ala Val Leu
                245                 250                 255

Gln Ser Ser Gly Leu Tyr Ser Leu Ser Ser Val Val Thr Val Pro Ser
            260                 265                 270

Ser Ser Leu Gly Thr Gln Thr Tyr Ile Cys Asn Val Asn His Lys Pro
        275                 280                 285

Ser Asn Thr Lys Val Asp Lys Lys Val Glu Pro Lys Ser Cys Asp Lys
    290                 295                 300

Thr His Thr Cys Pro Pro Cys Pro Ala Pro Glu Leu Leu Gly Gly Pro
305                 310                 315                 320

Ser Val Phe Leu Phe Pro Pro Lys Pro Lys Asp Thr Leu Met Ile Ser
                325                 330                 335

Arg Thr Pro Glu Val Thr Cys Val Val Val Asp Val Ser His Glu Asp
            340                 345                 350

Pro Glu Val Lys Phe Asn Trp Tyr Val Asp Gly Val Glu Val His Asn
        355                 360                 365

Ala Lys Thr Lys Pro Arg Glu Glu Gln Tyr Asn Ser Thr Tyr Arg Val
    370                 375                 380

Val Ser Val Leu Thr Val Leu His Gln Asp Trp Leu Asn Gly Lys Glu
385                 390                 395                 400

Tyr Lys Cys Lys Val Ser Asn Lys Ala Leu Pro Ala Pro Ile Glu Lys
                405                 410                 415

Thr Ile Ser Lys Ala Lys Gly Gln Pro Arg Glu Pro Gln Val Tyr Thr
            420                 425                 430

Leu Pro Pro Ser Arg Glu Glu Met Thr Lys Asn Gln Val Ser Leu Thr
        435                 440                 445

Cys Leu Val Lys Gly Phe Tyr Pro Ser Asp Ile Ala Val Glu Trp Glu
    450                 455                 460

Ser Asn Gly Gln Pro Glu Asn Asn Tyr Lys Thr Thr Pro Pro Val Leu
465                 470                 475                 480

Asp Ser Asp Gly Ser Phe Phe Leu Tyr Ser Lys Leu Thr Val Asp Lys
                485                 490                 495

Ser Arg Trp Gln Gln Gly Asn Val Phe Ser Cys Ser Val Met His Glu
            500                 505                 510

Ala Leu His Asn His Tyr Thr Gln Lys Ser Leu Ser Leu Ser Pro Gly
        515                 520                 525

Lys

<210>  127
<211>  292
<212>  PRT
<213>  Artificial sequence

<220>
<223>  Fynomer 2C1 linked to light chain of anti TNF antibody

<400>  127

Gly Val Thr Leu Phe Val Ala Leu Tyr Asp Tyr Lys Ala Phe Trp Pro
1               5                   10                  15

Gly Asp Ile Ser Phe His Lys Gly Glu Lys Phe Gln Ile Leu Arg Thr
            20                  25                  30

Ser Asp Gly Glu Trp Trp Ile Ala Arg Ser Leu Thr Thr Gly Glu Glu
        35                  40                  45

Gly Tyr Ile Pro Ser Asn Tyr Val Ala Pro Val Asp Ser Ile Gln Gly
    50                  55                  60

Gly Gly Gly Ser Gly Gly Gly Gly Ser Gly Gly Gly Gly Ser Asp Ile
65                  70                  75                  80

Gln Met Thr Gln Ser Pro Ser Ser Leu Ser Ala Ser Val Gly Asp Arg
                85                  90                  95

Val Thr Ile Thr Cys Arg Ala Ser Gln Gly Ile Arg Asn Tyr Leu Ala
            100                 105                 110

Trp Tyr Gln Gln Lys Pro Gly Lys Ala Pro Lys Leu Leu Ile Tyr Ala
        115                 120                 125

Ala Ser Thr Leu Gln Ser Gly Val Pro Ser Arg Phe Ser Gly Ser Gly
    130                 135                 140

Ser Gly Thr Asp Phe Thr Leu Thr Ile Ser Ser Leu Gln Pro Glu Asp
145                 150                 155                 160

Val Ala Thr Tyr Tyr Cys Gln Arg Tyr Asn Arg Ala Pro Tyr Thr Phe
                165                 170                 175

Gly Gln Gly Thr Lys Val Glu Ile Lys Arg Thr Val Ala Ala Pro Ser
            180                 185                 190

Val Phe Ile Phe Pro Pro Ser Asp Glu Gln Leu Lys Ser Gly Thr Ala
        195                 200                 205

Ser Val Val Cys Leu Leu Asn Asn Phe Tyr Pro Arg Glu Ala Lys Val
    210                 215                 220

Gln Trp Lys Val Asp Asn Ala Leu Gln Ser Gly Asn Ser Gln Glu Ser
225                 230                 235                 240

Val Thr Glu Gln Asp Ser Lys Asp Ser Thr Tyr Ser Leu Ser Ser Thr
                245                 250                 255

Leu Thr Leu Ser Lys Ala Asp Tyr Glu Lys His Lys Val Tyr Ala Cys
            260                 265                 270

Glu Val Thr His Gln Gly Leu Ser Ser Pro Val Thr Lys Ser Phe Asn
        275                 280                 285

Arg Gly Glu Cys
    290

## Description

The present invention relates to a fusion protein comprising or consisting of the antibody light chain consisting of SEQ ID NO: 1 and a polypeptide binding to IL-17, wherein said polypeptide binding to IL-17 is located downstream of the C-terminus of said antibody light chain within said fusion protein, and wherein said polypeptide consists of the amino acid sequence of SEQ ID NO: 2, wherein (a) one, two or three amino acids within amino acids positions 10 to 19 of SEQ ID NO: 2 is/are substituted, deleted or added, (b) one, two or three amino acids within amino acids positions 29 to 36 of SEQ ID NO: 2 is/are substituted, deleted or added, and/or (c) the amino acid position 48 is substituted or deleted, provided that the polypeptide binding to IL-17 has at least 85% sequence identity to the amino acid sequence of SEQ ID NO: 2, wherein the determination of identity excludes amino acid positions 12 to 17, 31 to 34 and 48 of SEQ ID NO: 2.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

CD4+ T cells play a central role in orchestrating immune responses by assisting other cells of the adaptive or innate immune system. In early studies two classes of CD4+ T cells (Th1 and Th2) were identified. More recently, a new subset of CD4+ T cells, the Th17 lineage was identified. Th17 cells appear to have evolved as a branch of the adaptive immune system specialized in enhanced host protection against extracellular bacteria as well as some fungi and microbes not well covered by Th1 or Th2 immunity.

Th17 cells were identified in the context of the discovery of a new cytokine family, the IL-17 family, which is presently known to comprise six members (IL-17A-F). IL-17 (previously named CTLA-8) is mainly expressed by Th17 cells and was designated IL-17A to indicate that it is the founding member of this cytokine family. IL-17 members share no sequence homology with other presently known mammalian proteins and therefore constitute a distinct cytokine family. Structural features of IL-17 family members deduced from the crystal structure of IL-17F suggest that, similar to many cytokines, each of the family members is probably produced as a homodimer, although structural similarities imply that heterodimers may exist. Very recently, a heterodimer of IL-17A and IL-17F expressed by activated human CD4+ T cells was identified that signals through the IL-17RA/IL-17RC complex (Wright J.F. et al. (2008) J. of Immunol., 181, p. 2799-2805).

The identification of Th17 cells as central mediators in chronic inflammatory processses and as principal pathogenic effectors in several types of autoimmunity conditions previously thought to be Th1-mediated promises new therapeutic approaches (Weaver T. et al. (2008) Annu. Rev. Immunol., 25, p. 821-852). Indeed, the pro-inflammatory cytokine IL-17 is mainly expressed by Th17 cells and is present at elevated levels in synovial fluid of patients with rheumatoid arthritis (RA) and has been shown to be involved in early RA development. In addition, IL-17 is a potent inducer of TNF-alpha and IL-1, the latter being mainly responsible for bone erosion and the very painful consequences for affected patients (Lubberts E. (2008) Cytokine, 41, p. 84-91). Furthermore, inappropriate or excessive production of IL-17 is associated with the pathology of various other diseases and disorders, such as osteoarthritis, loosening of bone implants, acute transplant rejection (Antonysamy et al. (1999) J. Immunol, 162, p. 577-584; van Kooten et al. (1998) J. Am. Soc. Nephrol., 9, p.1526-1534), septicemia, septic or endotoxic shock, allergies, asthma (Molet et al. (2001) J. Allergy Clin. Immunol., 108, p. 430-438), bone loss, psoriasis (Teunissen et al. (1998) J. Invest. Dermatol, 111, p. 645-649), ischemia, systemic sclerosis (Kurasawa et al. (2000) Arthritis Rheum., 43, p. 2455-2463), stroke, and other inflammatory disorders.

Consequently, anti-IL-17 compounds have potential as anti-inflammatory agents, a therapeutic approach in line with a number of *in vivo* studies demonstrating that IL-17 neutralization reduces inflammatory processes such as arthritis. For example, the early neutralization of endogenous IL-17 by an IL-17 receptor-IgG1-Fc fusion protein starting after the immunization protocol during the initial phase of arthritis suppresses the onset of experimental arthritis (Lubberts et al. (2001) J. Immunol., 167, p. 1004-1013). Moreover, treatment with a neutralizing anti-IL-17 antibody in an animal model after the onset of collagen-induced arthritis reduced joint inflammation, cartilage destruction and bone erosion (Lubberts et al. (2004) Arthritis and Rheumatism, 50; 650-659). Histological analysis confirmed the suppression of joint inflammation, and systemic IL-6 levels were significantly decreased after treatment with an anti-IL-17 antibody. In contrast, systemic as well as local IL-17 overexpression using an adeno-viral vector expressing murine IL-17 accelerated the onset of collagen-induced arthritis (CIA) and aggravated synovial inflammation at the site (Lubberts et al. (2001) J. Immunol.,167, p. 1004-1013 and Lubberts et al. (2002) Inflamm. Res. 51, p102-104). Recently, it has been reported that the treatment with an anti-IL-17A monoclonal antibody induced clinically relevant responses in patients suffering from immune-mediated diseases such as psoriasis, rheumatoid arthritis and uveitis (Hueber et al. (2010) Sci Transl Med. 2(52):52ra72).

In addition to antibodies, non-immunoglobulin-derived binding reagents (collectively designated "scaffolds"; see, for example, Skerra (2000) J. Mol. Recognit. 13, 167-187) have been suggested for use as diagnostic and therapeutic agents. More than 50 different protein scaffolds have been proposed over the past 10 to 15 years, the most advanced approaches in this field being (as summarized in Gebauer and Skerra (2009) Curr Opinion in Chemical Biology 13:245-255): affibodies (based on the Z-domain of staphylococcal protein A), Kunitz type domains, adnectins (based on the 10th domain of human fibronectin), anticalins (derived from lipocalins), DARPins (derived from ankyrin repeat proteins), avimers (based on multimerized LDLR-A), affitins (based on Sac7d from the hyperthermophilic archaeon), and Fynomers, which are derived from the human Fyn SH3 domain.

In general, SH3 domains are present in a large variety of proteins participating in cellular signal transduction (Musacchio et al. (1994) Prog. Biophys. Mol. Biol. 61; 283-297). These domains do not occupy a fixed position within proteins and can be expressed and purified independently. More than 1000 occurrences of the domain are presently known including about 300 human SH3 domains (Musacchio (2003) Advances in Protein Chemistry. 61; 211-268). Although there is great sequence diversity among SH3 domains, they all share a conserved fold: a compact beta barrel formed by two anti-parallel beta-sheets (Musacchio (2003) Advances in Protein Chemistry. 61; 211-268). Typically, SH3 domains bind to proline-rich peptides containing a PXXP core-binding motif (Ren et al. (1993) Science 259; 1157-1161), but examples of unconventional SH3 binding sites have also been described (Karkkainen et al. (2006) EMBO Rep. 7;186-191). Most of the SH3 domains sequenced so far have an overall length of approximately 60 to 65 amino acids, but some of them may feature as many as 85 amino acids due to inserts into the loops connecting the main conservative elements of the secondary structure (Koyama et al. (1993) Cell 72(6); 945-952). An alignment of different SH3 domains revealed conserved amino acid residues responsible for the proper structure formation as well as for the canonical proline-rich motif recognition (Larson et al. (2000) Protein Science 9; 2170-2180).

The SH3 domain of the Fyn kinase (Fyn SH3) comprises 63 residues (aa 83-145 of the sequence reported by Semba et al. (1986) (Proc. Natl. Acad. Sci. U S A 83(15): 5459-63) and Kawakami et al. (1986) (Mol Cell Biol. 6(12): 4195-201). Fyn is a 59 kDa member of the Src family of tyrosine kinases. As a result of alternative splicing the Fyn protein exists in two different isoforms differing in their kinase domains: one form is found in thymocytes, splenocytes and some hematolymphoid cell lines, while a second form accumulates principally in brain (Cooke and Perlmutter (1989),New Biol. 1(1): 66-74). The biological functions of Fyn are diverse and include signalling via the T cell receptor, regulation of brain function as well as adhesion mediated signalling (Resh (1998) Int. J. Biochem. Cell Biol. 30(11): 1159-62). It is an intracellular protein. SEQ ID NO: 2 shows the Fyn SH3 sequence (aa 83-145 of Fyn kinase as reported by Kawakami et al. and Semba et al. in 1986, see above):
GVTLFVALYDYEARTEDDLSFHKGEKFQILNSSEGDWWEARSLTTGETGYIPSNYVAPVDSIQ (SEQ ID NO: 2)

The sequence of the RT-Src and the n-Src loop are underlined and double-underlined, respectively.

The amino acid sequence of Fyn SH3 is fully conserved among man, mouse, rat and monkey (gibbon). Chicken Fyn SH3 differs in one, the one of *Xenopus leavis* in two amino acid positions from the corresponding human domain. Just as other SH3 domains the Fyn SH3 is composed of two antiparallel β-sheets and contains two flexible loops (called RT-Src and n-Src-loops) in order to interact with other proteins.

Earlier the present inventors demonstrated that the Fyn SH3 domain is a particularly attractive scaffold for the generation of binding proteins ("Fynomers") because it (i) can be expressed in bacteria in soluble form in high amounts, (ii) is monomeric and does not aggregate when stored in solution, (iii) is very stable (Tₘ 70.5 °C), (iv) lacks cysteine residues, and (v) is of human origin featuring an amino acid sequence completely conserved from mouse to man and, hence, non-immunogenic (Grabulovski WO 2008/022759; Grabulovski et al. (2007) JBC, 282, p. 3196-3204).

Further IL-17A binding molecules, in particular Fynomers with high specificity and high affinity for IL-17A have been disclosed in Grabulovski et al., WO 2011/023685.

A further key molecule involved in the etiology of inflammatory diseases, autoimmune and bone-loss related diseases, in particular in the various forms of arthritis including rheumatoid arthritis is tumor necrosis factor (TNF). Koenders et al. (2011) (Arthritis and Rheumatism, 63; 2329-2339) describe that the interplay between tumor necrosis factor and IL-17 trigger molecular mechanisms leading to irreversibly cartilage destruction in an animal model of arthritis. Moreover, it has been found that the combination of a soluble interleukin-17 receptor and a tumor necrosis factor binding protein was more effective in the treatment of arthritis than either anti-cytokine treatment alone. The TNF binding molecule described in the publication of Koenders et al. (2011) is a dimerically linked PEGylated soluble p55 TNF receptor I. Other TNF binding molecules, designed for therapeutic applications, are described in Tak and Kalden (2011) (Arthritis Research and Therapy, 13; 1-14). WO 2010/102251 describes binding proteins comprising first and second polypeptide chains, wherein the binding protein is capable of binding human IL-17 and TNF. Both polypeptide chains have a molecular architecture formed by antibody variable and constant domains.

In view of the prior art as discussed above, the technical problem underlying the present invention can be seen in the provision of improved or alternative means and methods for treating or preventing disorders where IL-17 and TNF are involved, such disorders including inflammatory, autoimmune and bone-loss related diseases.

This problem has been solved by the subject-matter of the claims.

Accordingly, the present invention, in a first aspect, relates to a fusion protein comprising or consisting of the antibody light chain consisting of SEQ ID NO: 1 and a polypeptide binding to IL-17, wherein said polypeptide binding to IL-17 is located downstream of the C-terminus of said antibody light chain within said fusion protein, and wherein said polypeptide consists of the amino acid sequence of SEQ ID NO: 2, wherein (a) one, two or three amino acids within amino acids positions 10 to 19 of SEQ ID NO: 2 is/are substituted, deleted or added, (b) one, two or three amino acids within amino acids positions 29 to 36 of SEQ ID NO: 2 is/are substituted, deleted or added, and/or (c) the amino acid position 48 is substituted or deleted, provided that the polypeptide binding to IL-17 has at least 85% sequence identity to the amino acid sequence of SEQ ID NO: 2, wherein the determination of identity excludes amino acid positions 12 to 17, 31 to 34 and 48 of SEQ ID NO: 2.

The term "fusion protein" refers to a protein, preferably a polypeptide, comprising or consisting of the sequences of SEQ ID NOs: 1 and 2.

As is well-known in the art, when two polypeptides or functional polypeptide domains are to be combined in a single construct, numerous options are available, said options including N-terminal fusion, C-terminal fusion, said fusions being with or without linker sequences, insertions of one of the polypeptides into loop regions of the other polypeptide, and the like. Typically, the expression yield as well as the functional properties of the bifunctional construct depend significantly on the particular architecture chosen. In many instances, attempts to create a functional combined construct fail altogether. Also the present inventors experienced failures, said failures including low or no expression and unsatisfactory pharmacological properties. The specific molecular architecture of the main embodiment surprisingly provides for (i) good expression, (ii) function, i.e., the capability of bispecific constructs according to the present invention (see below for further details) to bind both IL-17 and TNF, and (iii) favorable pharmacokinetic properties, namely in particular a very advantageous long half-life in vivo.

The term polypeptide refers to a single molecule which molecule is a polycondensate of amino acids, the amino acids being linked by main chain peptide bonds. To the extent the main embodiment refers to a polypeptide consisting of SEQ ID NOs: 1 and 2, these two sequences may be directly fused to each other which is also subject of a preferred embodiment discussed further below. To the extent the main embodiment refers to a polypeptide comprising the sequences of SEQ ID NOs: 1 and 2, it is understood that between the sequences of SEQ ID NOs: 1 and 2, upstream of SEQ ID NO: 1 and/or downstream of SEQ ID NO: 2 further flanking or linking amino acid sequences may be present which are not further limited in sequence or size. Preferably, these flanking sequences do not exceed a length of 100, 50, 40, 30, 25, 20 or 15 amino acids.

In either case, it is understood that not the sequence of SEQ ID NO: 2 as such is comprised in polypeptides or proteins according to the invention, but instead the modified version thereof as defined in the main embodiment, said modified version deviating from the sequence of SEQ ID NO: 2 by the defined substitution(s), deletion(s) and/or addition(s).

The term "protein" as used herein embraces polypeptides as well as oligomers formed from said polypeptides. Oligomer formation typically involves non-covalent attachment. According to the invention, such oligomers are homooligomers and are preferably selected from dimers, trimers and tetramers.

According to the invention, the sequence of SEQ ID NO: 2 is located downstream of the C-terminus of the sequence of SEQ ID NO: 1. This arrangement may be implemented by either directly fusing the C-terminus of SEQ ID NO: 1 to the N-terminus of SEQ ID NO: 2, more specifically by the formation of a main chain peptide bond between the carboxy group of the C-terminal amino acid of SEQ ID NO: 1 and the amino group of the N-terminal amino acid of SEQ ID NO: 2. Alternatively, there may be an intervening linker sequence which is the subject of a preferred embodiment as discussed further below.

For the purpose of expression, there may be an N-terminal flanking sequence or "leader sequence", which is also referred to as "signal peptide" in the art. The skilled person can choose suitable leader sequences without further ado. A leader sequence is preferably employed for the expression of any antibody chain (including light chain, heavy chain) or domain and is dispensable in the mature construct.

A preferred N-terminal flanking sequence of the sequence of SEQ ID NO: 1 is the leader sequence of SEQ ID NO: 123. In the alternative, either no or any other suitable leader sequence may be used. In the fusion protein and in the bispecific construct (see below), when intended for medical application, a leader sequence is preferably not present.

A particularly preferred fusion protein according to the present invention consists of the sequence set forth in SEQ ID NO: 124. The sequence of SEQ ID NO: 124 is a single contiguous polypeptide chain connected by main chain peptide bonds. It consists of the following sequences, ordered from the N- to the C-terminus: SEQ ID NOs: 1, 120, and 109. While this is particularly preferred, any other linker sequence, and optionally a leader sequence as disclosed herein or at the skilled person's disposal may be used such that constructs of the following architecture, from N- to C-terminus, are obtained: optional leader sequence - SEQ ID NO: 1 - linker sequence - variants of SEQ ID NO: 2 as defined in the main embodiment, wherein among said variants preference is given to the sequence consisting of SEQ ID NO: 109, said sequence SEQ ID NO: 109 being also designated "2C1" herein. As previously noted, leader sequence and/or linker sequence may be dispensed with.

The upstream component of the fusion protein according to the main embodiment is characterized in terms of its structure, namely in that it comprises or consists of SEQ ID NO: 1. SEQ ID NO: 1 is inherently an antibody light chain, more specifically the light chain of an anti TNF antibody.

The downstream component of the fusion protein according to the present invention comprises or consists of the sequence of SEQ ID NO: 2, provided that the sequence of the recited polypeptide binding to IL-17 differs from the sequence of SEQ ID NO: 2 as required by the main embodiment. Preferred embodiments of said variants of SEQ ID NO: 2 are described in WO 2011/023685, and particularly preferred variants are the subject of a preferred embodiment discussed herein below. Variants of SEQ ID NO: 2 of the present invention are also referred to as "Fynomers". Said Fynomers are designed to bind interleukin-17 (IL-17). Interleukin-17 is a cytokine known in the art and described in publications as cited herein above. A preferred IL-17 family member is IL-17A. The disclosed Fynomers bind IL-17A.

The recited at least 85% sequence identity embrace any higher value of sequence identity. Envisaged values of sequence identity are at least 86%, at least 87%, at least 88%, at least 89% at least 90%, at least 91%, at least 92%, at least 93%, at least 94%, at least 95%, at least 96%, at least 97%, at least 98%, at least 99% and 100%. When determining sequence identity, the indicated amino acid positions (12 to 17, 31 to 34 and 48), which positions are defined in relation to the sequence consisting of SEQ ID NO: 2, are to be disregarded. In other terms, while items (a), (b) and (c) of the main embodiment require differences with regard to the wild type SH3 domain sequence of SEQ ID NO: 2, the remainder of the Fynomer sequence has a sequence identity to the wild-type between 85% and 100%. Determining the such defined sequence identity can be done, for example, by performing a pair wise sequence alignment, disregarding the sequence identity reported by the alignment program and re-evaluating the degree of sequence identity such that positions 12 to 17, 31 to 34 and 48 of SEQ ID NO: 2 are not considered. Such re-evaluation of sequence identity can be done by the skilled person without further ado in view of these instructions. The ranges from 12 to 17 and 31 to 34 are underlined in the presentation of the sequence of SEQ ID NO: 2 in the background section herein above. When including two flanking amino acids at either side, the ranges from 10 to 19 and 29 to 36 (always in terms of amino acids of the sequence consisting of SEQ ID NO: 2) are obtained, which are the regions according to items (a) to (b) of the main embodiment where, in either case, one, two or three amino acid substitutions, deletions or additions are to be effected.

As used herein, the term "amino acid sequence identity" between amino acid sequences is meant to relate to the common and widely used alignment and comparison techniques of the person skilled in biochemistry. The amino acid sequence identity of two amino acid sequences can be determined by common alignment methods and tools. For example, for determining the extent of an amino acid sequence identity of an arbitrary polypeptide relative to the amino acid sequence of SEQ ID NO: 2, the SIM Local similarity program can be employed (Xiaoquin Huang and Webb Miller (1991), Advances in Applied Mathematics, vol. 12: 337-357), that is freely available from the authors and their institute (see also the world wide web: http://www.expasy.org/tools/sim-prot.html). For multiple alignment analysis ClustalW can be used (Thompson et al. (1994) Nucleic Acids Res., 22(22): 4673-4680). The extent of the amino acid sequence identity of a polypeptide to the amino acid sequence of SEQ ID NO: 2 is determined relative to the complete sequence of SEQ ID NO: 2, with the exception of positions 12 to 17, 31 to 34 and 48 of SEQ ID NO: 2.

A substitution may be a conservative or a non-conservative substitution, but preferably is a conservative substitution. In some embodiments, a substitution also includes the exchange of a naturally occurring amino acid with a non-naturally occurring amino acid. A conservative substitution comprises the substitution of an amino acid with another amino acid having a chemical property similar to the amino acid that is substituted. Preferably, the conservative substitution is a substitution selected from the group consisting of: (i) a substitution of a basic amino acid with a different basic amino acid; (ii) a substitution of an acidic amino acid with a different acidic amino acid; (iii) a substitution of an aromatic amino acid with a different aromatic amino acid; (iv) a substitution of a non-polar, aliphatic amino acid with a different non-polar, aliphatic amino acid; and (v) a substitution of a polar, uncharged amino acid with a different polar, uncharged amino acid. A basic amino acid is selected from the group consisting of arginine, histidine, and lysine. An acidic amino acid is selected from aspartate or glutamate. An aromatic amino acid is selected from the group consisting of phenylalanine, tyrosine and tryptophane. A non-polar, aliphatic amino acid is selected from the group consisting of glycine, alanine, valine, leucine, methionine and isoleucine. A polar, uncharged amino acid is selected from the group consisting of serine, threonine, cysteine, proline, asparagine and glutamine. In contrast to a conservative amino acid substitution, a non-conservative amino acid substitution is the exchange of one amino acid with any amino acid that does not fall under the above-outlined conservative substitutions (i) through (v).

Polypeptides and fusion proteins of the invention may be prepared by any of the many conventional and well known techniques such as plain organic synthetic strategies, solid phase-assisted synthesis techniques or by commercially available automated synthesizers. On the other hand, they may also be prepared by conventional recombinant techniques alone or in combination with conventional synthetic techniques.

A fusion protein according to the present invention, when combined with an antibody heavy chain, said antibody heavy chain being chosen such that upon assembly of an antibody or an antibody-like molecule, an antigen binding site capable of binding, preferably specifically binding, TNF is formed, provides a novel means for treating or preventing diseases or disorders in the etiology of which IL-17 and TNF are involved, such diseases being further defined below. TNF is sometimes also referred to as "TNF-α".

The present inventors surprisingly found that it is the particular choice of the fusion site which has a significant influence on (i) the capability of the obtained fusion protein to be expressed, and (ii) the pharmacokinetics of the fusion protein, in particular the half-life in serum. As it is apparent from the examples enclosed herewith, two rather similar constructs differ dramatically in terms of serum half-life. A further construct delivered low or no expression; see the construct designated "2C1LCHC" as described in the Examples. Two expressible constructs, but with different half lifes, are herein designated HC2C1 and LC2C1. LC2C1 falls under the terms of the claims. The difference between the two is the fusion site of said polypeptide binding to IL-17, which in case of HC2C1 is the C-terminus of the heavy chain of the anti-TNF antibody and in case of LC2C1 is the C-terminus of the light chain of the anti-TNF antibody. The plasma half-life of LC2C1 as compared to HC2C1 is about as twice as long. Accordingly, the surprising effect observed by the present inventors in terms of increased half-life directly translates its significant benefit for the patient since less active agent is to be administered and/or longer intervals between successive treatments become possible. Last but not least, the public health system benefits from lower treatment costs.

In a preferred embodiment, said IL-17 binding polypeptide consists of an amino acid sequence selected from the group consisting of SEQ ID NOs: 109, 3 to 108 and 110 to 118.

The sequences of SEQ ID NOs: 3 to 118 are identical to the sequences of SEQ ID NO: 1 to 116 as disclosed in WO 2011/023685.

In the context of the present invention, particular preference is given to the Fynomer consisting of SEQ ID NO: 109, also designated "2C1" herein as well as in WO 2011/023685. A bispecific construct, the term bispecific construct being defined further below, comprising a fusion protein according to the present invention, said fusion protein comprising the Fynomer consisting of SEQ ID NO: 109 has been analyzed in detail in the enclosed Examples.

In a further preferred embodiment, said polypeptide, i.e. the polypeptide binding to IL-17, is fused to the C-terminus of the antibody light chain. This preferred embodiment requires that the carboxy group of the C-terminal amino acid of the sequence consisting of SEQ ID NO: 1 is connected via a main chain peptide bond to the amino group of the N-terminal amino acid of a variant of SEQ ID NO: 2, the variant being as defined in the main embodiment. In other words, there is no intervening linker sequence between the sequences of SEQ ID NO: 1 and the variant of SEQ ID NO: 2. Yet in other terms, these two sequences can be referred to as being "directly" fused to each other in the required orientation. In an alternative preferred embodiment, said polypeptide, i.e. the polypeptide binding to IL-17, is connected to the C-terminus of the antibody light chain via a linker.

Suitable linkers are at the skilled person's disposal. Preference is given to peptidic linkers, more specifically to oligopeptides having a length from 2 to 30 amino acids. Use of a single amino acid is also deliberately envisaged. Preferred length ranges are from 5 to 15 amino acids. Other preferred lengths are 3, 4, 6, 7, 8, 9, 10, 11, 12, 13, 14, 16, 17, 18, 19 or 20 amino acids.

Particularly preferred are linkers which are peptides which consist of at least 50%, at least 60%, at least 70%, at least 80%, at least 90% or 100% small amino acids such as glycine, serine and alanine. Particularly preferred are linkers consisting of glycines and serines only. Most preferred are the linkers of SEQ ID NOs: 120 to 122 with special preference given to a linker being a peptide consisting of the sequence of SEQ ID NO: 120.

In a second aspect, the present invention provides a bispecific construct comprising or consisting of at least one copy, preferably two copies of the fusion protein of the invention and at least one copy, preferably two copies of the antibody heavy chain of SEQ ID NO: 119.

The term "bispecific" has a meaning as established in the art. It refers to a construct which is capable of concomitantly binding two target molecules, in the present case said target molecules being interleukin-17 on the one hand and TNF on the other hand. According to the invention, such bispecific construct comprises or consists of at least one, preferably two copies of the fusion protein according to the invention and at least one, preferably two copies of the antibody heavy chain consisting of SEQ ID NO: 119. The fusion protein *per se* comprises a functional IL-17 binding site owing to the presence of a Fynomer. Said antibody heavy chain is the heavy chain of an anti TNF antibody. The light chain component of the antibody as comprised in the fusion protein, when brought together with an antibody heavy chain, provides for the formation of an antigen binding site which site recognizes TNF.

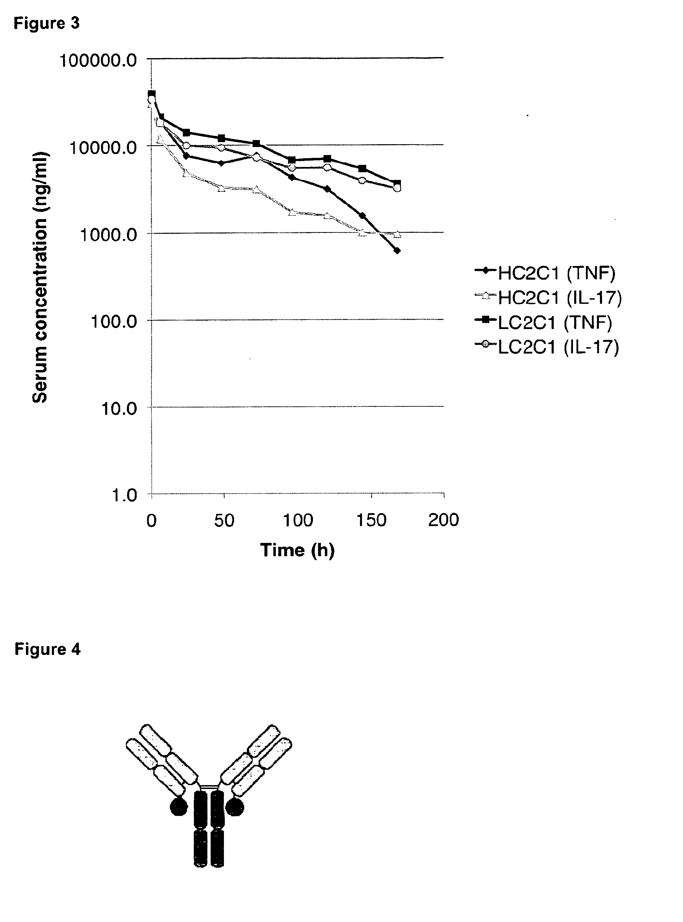

Particular preference is given to a bispecific construct which comprises or consists of two copies of a fusion protein according to the invention and two copies of said heavy chain. Within said fusion protein, particular preference is given to a fusion protein consisting of the sequence of SEQ ID NO: 124 which in turn has the substructure as discussed herein above. In particular, it comprises the preferred Fynomer consisting of the sequence of SEQ ID NO: 109. The term "bispecific" does not limit the number of binding sites for each target within a single bispecifc construct. As shown in Figure 4, there may be two IL-17 binding sites and two TNF binding sites. The construct of Figure 4 is accordingly bivalent with regard to both IL-17 and TNF.

Since the fusion protein according to the invention comprises a light chain of an antibody, it necessarily comprises also the variably domain of said light chain. Similarly, since the bispecific construct according to the present invention requires an antibody heavy chain, it necessarily also comprises a variable domain within said heavy chain. Light chain variable domain and heavy chain variable domain together form an antigen binding site, wherein said antigen binding site binds to, preferably specifically binds to TNF. The term "specifically binds to" refers to those cases where TNF is bound with equilibrium binding constant K_{d} which is by a factor 2 smaller, preferably by a factor 5 or a factor 10 smaller as compared to the equilibrium binding constant observed for the binding of said bispecific construct to an unrelated protein, such unrelated protein being not a member of the TNF family.

A particularly preferred architecture of the bispecific construct according to the present invention is displayed in Figure 4 as enclosed herewith. Such bispecific construct consists of two copies of the fusion protein according to the present invention and two copies of the mentioned antibody heavy chain.

A bispecific construct according to the present invention may be obtained by bringing together, under suitable conditions, said fusion protein and said antibody heavy chain. The skilled person is aware of suitable conditions. Such bringing together under suitable conditions provides for the non-covalent assembly triggered by the interactions between the antibody light chain as comprised in said fusion protein and said antibody heavy chain, eventually giving rise to a molecular architecture such as the one displayed in Figure 4. Preferably, disulfide bonds as they are commonly found in antibodies are present in said bispecific construct; see the exemplary drawing in Figure 4. Disulfide bonds are typically present in the proximity of the hinge region and connect two heavy chains and/or a light chain and a heavy chain.

While the exemplary construct shown in Figure 4 comprises four polypeptide chains, it is also envisaged to use bispecific constructs comprising two polypeptide chains, namely one fusion protein according to the present invention as first polypeptide chain, said fusion protein being a single chain polypeptide, and one antibody heavy chain as second polypeptide chain.

The present invention furthermore relates to yet further bispecific constructs which further bispecific constructs may be devoid of one or more of the constant domains of the said heavy chain.

In a further aspect, the present invention relates to a pharmaceutical composition comprising the fusion construct according to the present invention or the bispecific construct according to the present invention.

It is preferred that within said pharmaceutical composition said bispecific construct is the only active agent. On the other hand, it is envisaged to include also further pharmaceutically active agents.

Pharmaceutical compositions of the invention may be manufactured in any conventional manner. In effecting treatment of a subject suffering from the diseases indicated below, at least one compound of the present invention can be administered in any form or mode which makes the active agent bioavailable in an effective amount, including oral or parenteral routes. For example, compositions of the present invention can be administered subcutaneously, intramuscularly, intravenously, intraperitoneally, by inhalation and the like.

Preference is given to subcutaneous injection, preferably weekly or biweekly or once per month.

One skilled in the art in the field of preparing formulations can readily select the proper form and mode of administration depending upon the particular characteristics of the product selected, the disease or condition to be treated, the stage of the disease or condition and other relevant circumstances (see. e.g. Remington's Pharmaceutical Sciences, Mack Publishing Co. (1990)).

A suitable carrier or excipient may be a liquid material which can serve as a vehicle or medium for the active ingredient. Suitable carriers or excipients are well known in the art and include, for example, stabilizers, antioxidants, pH-regulating substances, controlled-release excipients, etc. A composition according to the invention may be provided in lyophilized form. For immediate administration it is dissolved in a suitable aqueous carrier, for example sterile water for injection or sterile buffered physiological saline. If it is desirable to produce larger volumes for administration by infusion rather than as a bolus injection, it is advantageous to incorporate human serum albumin or the patient's own heparinised blood into the solvent at the time of final formulation. Alternatively, the formulation can be administered subcutaneously. The presence of an excess of a physiologically inert protein such as human serum albumin prevents loss of the pharmaceutically effective polypeptide by adsorption onto the walls of the container and tubing used for the infusion solution. If albumin is used, a suitable concentration is from 0.5 to 4.5% by weight of the saline solution.

The amount and mode of administration of the inventive compounds, i.e., fusion proteins, bispecific constructs, nucleic acids, vectors, and isolated cells for treating and/or preventing a disease or medical condition, will, of course, vary depending upon the particular compound of the invention, the individual patient group or patient, the presence of further medically active compounds and the nature and severity of the condition being treated.

However, it is presently preferred that for prophylactic and/or therapeutic use dosages of about 0.01 mg to about 20 mg per kilogram body weight, preferably about 0.1 mg to about 5 mg per kilogram body weight should be administered.

Preferably, the frequency of administration for prophylactic and/or therapeutic uses lies in the range of about twice per week up to about once every 3 months, preferably about weekly up to about once every 10 weeks, more preferably once a week up to once per month .

In a preferred embodiment, said pharmaceutical composition is for use in a method of treating an inflammatory, autoimmune and/or bone loss related disease.

In a preferred embodiment, said disease is selected from the group consisting of arthritis, preferably rheumatoid arthritis, arthritis chronica progrediente, reactive arthritis, psoriatic arthritis, enterophathic arthritis and arthritis deformans, rheumatic diseases, spondyloarthropathies, ankylosing spondylitis, Reiter syndrome, hypersensitivity (including both airways hypersensitivity and dermal hypersensitivity), allergies, systemic lupus erythematosus, inflammatory muscle disorders, polychondritis, sclerodoma, Wegener granulomatosis, dermatomyositis, Steven-Johnson syndrome, chronic active hepatitis, myasthenia gravis, psoriasis, idiopathic sprue, autoimmune inflammatory bowel disease, ulcerative colitis, Crohn's disease, Irritable Bowel Syndrome, endocrine ophthalmopathy, Graves disease, sarcoidosis, multiple sclerosis, primary biliary cirrhosis, juvenile diabetes (diabetes mellitus type I), autoimmune haematological disorders, hemolytic anaemia, aplastic anaemia, pure red cell anaemia, idiopathic thrombocytopenia, uveitis (anterior and posterior), keratoconjunctivitis sicca, vernal keratoconjunctivitis, interstitial lung fibrosis, glomerulonephritis (with and without nephrotic syndrome), idiopathic nephrotic syndrome or minimal change nephropathy, tumors, inflammatory disease of skin, cornea inflammation, myositis, loosening of bone implants, metabolic disorders, atherosclerosis, diabetes, and dislipidemia, bone loss, osteoarthritis, osteoporosis, periodontal disease of obstructive or inflammatory airways diseases asthma, bronchitis, pneumoconiosis, pulmonary emphysema, acute and hyperacute inflammatory reactions, diseases involving IL-17A-mediated TNF-alpha, acute infections, septic shock, endotoxic shock, adult respiratory distress syndrome, meningitis, pneumonia, severe burns, cachexia wasting syndrome, stroke, herpetic stromal keratitis and dry eye disease.

In a fourth aspect, the present invention furthermore provides a nucleic acid molecule encoding the fusion protein as defined herein above or to at least one nucleic acid molecule encoding the bispecific construct according to the present invention.

Since the bispecific construct may be a complex of one (or more) copies of two distinct molecules, the constituents of the bispecific construct may be encoded by either one or two (or more than two) nucleic acid molecules.

In a yet further aspect, the present invention provides at least one vector comprising said nucleic acid molecule(s).

Nucleic acids of the invention can be DNA, RNA, PNA and any other analogues thereof. The vectors and isolated cells, in particular host cells, may be any conventional type that suits the purpose, e.g. production of polypeptides and/or fusion proteins of the invention, therapeutically useful vectors and isolated cells, e.g. for gene therapy. The skilled person will be able to select those nucleic acids, vectors and isolated cells from an abundant prior art and confirm their particular suitability for the desired purpose by routine methods and without undue burden.

Preferably the nucleic acid is operably linked to a promoter, preferably linked to a promoter selected from the group of prokaryotic promoters consisting of T5 promoter/lac operator element, T7 promotor/lac operator element, or from the group of eukaryotic promoters consisting of hEF1-HTLV, CMV enh/hFerL promoter.

It is also preferred that a vector of the invention is one comprising a nucleic acid of the invention and preferably being capable of producing a polypeptide or fusion protein of the invention. Preferably such a vector is selected from the group consisting of pQE vectors, pET vectors , pFUSE vectors, pUC vectors, YAC vectors, phagemid vectors, phage vectors, vectors used for gene therapy such as retroviruses, adenoviruses, adeno-associated viruses.

In a further aspect, provided is an isolated cell comprising the nucleic acid molecule(s) as defined herein above and/or the vector(s) as defined herein above. An isolated cell as recited herein may be a cell in vitro, a cell in culture or a host cell.

In a yet further aspect, the present invention provides a method of producing the fusion protein according to the invention or the bispecific construct according to the invention, which method comprises (a) culturing the above defined isolated cells and (b) isolating the produced fusion protein or bispecific construct.

Preferred isolated cells are host cells including CHO cells. Conditions for culturing may be chosen depending on the particular host cells used which can be done by the skilled person without further ado. Isolation of the desired product, i.e. said fusion protein or said bispecific construct, can be effected by purification methods, preceded by breaking up said isolated cells. Suitable methods are well-known in the art and at the skilled person's disposal.

The Figures show:
- **Figure 1:**: Size exclusion chromatograms (SEC) of: A) HC2C1; and B) LC2C1. For designations see Example 1.
- **Figure 2:**: Results of an IL-17A/TNF inhibition cell assay using Normal Human Dermal Fibroblasts. A comparable dose-dependent inhibition of IL-17A/TNF-induced IL-6 production in NHDF cells is observed for both HC2C1 and LC2C1.
- **Figure 3:**: Pharmacokinetic data of HC2C1 and LC2C1 in mice: serum concentrations in serum are plotted versus time after intravenous injection in a semi-logarithmic display.
- **Figure 4:**: Schematic drawing of LC2C1.

The Examples illustrate the invention.

### Example 1

### Expression and purification yields

HC2C1 (bispecific construct (not according to the invention) with heavy chain and light chain consisting of the sequences of SEQ ID NOs: 125 and 1, respectively) and LC2C1 (bispecific construct (according to the invention) with heavy chain and light chain consisting of the sequences of SEQ ID NOs: 119 and 124, respectively) were transiently transfected into FreeStyle CHO-S cells and expressed in serum-free/animal component-free media for 6-10 days. 2C1LCHC is a bispecific construct comprising fusion proteins (not according to the invention) wherein a Fynomer is connected to the N-terminus of both the antibody light chain and the antibody heavy chain. The proteins were purified from the supernatants by Protein A affinity chromatography (Mab Select Sure column; GE Healthcare) and by size exclusion chromatography (SEC; Superdex G200, 30/100 GL column; GE Healthcare) on an Akta Purifier instrument (GE Healthcare). Concentrations were determined by absorbance measurements at 280 nm. Yields are listed in Table 1.

**Table 1: Purification yields**

| Construct | SEQ ID NOs (heavy chain, light chain) | Yield (mg/L) |
|---|---|---|
| HC2C1 | 125, 1 | 80 |
| LC2C1 | 119, 124 | 103 |
| 2C1LCHC | 126, 127 | 7 |

### Example 2:

### Analytical size exclusion chromatography (SEC)

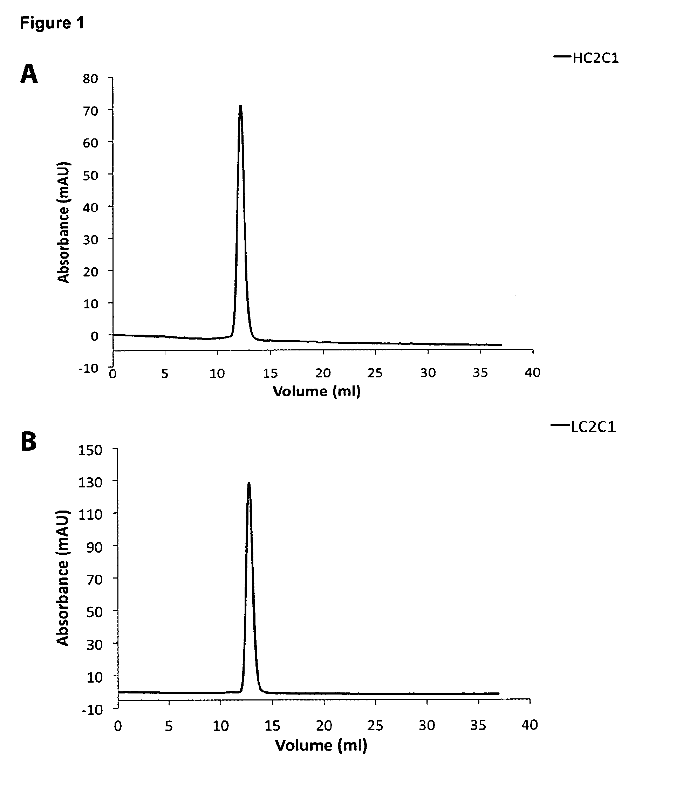

The oligomeric state of the purified proteins HC2C1 and LC2C1 was assessed by analytical SEC on an Akta Purifier using a Superdex G200, 30/100 GL column (GE Healthcare). SEC profiles demonstrated that both constructs HC2C1 and LC2C1 eluted as single monomeric peaks at the expected retention volumes for proteins with a molecular weight of about 160 kDa (see Fig. 1 A and B). No aggregates or proteolysis products could be observed. 2C1LCHC failed to express at significant levels.

### Example 3:

### Affinity measurements

The affinities of HC2C1 and LC2C1 to IL-17A (human and cynomolgus) and to TNF (human and cynomolgus) were measured using a BlAcore T200 instrument (GE Healthcare). A Series S CM5 chip (GE Healthcare) was coated with 8000 RU goat anti-human IgG Fc-specific antibody (Jackson Immunoresearch). The running buffer was PBS containing 0.05% Tween 20. The interactions were measured by capturing about 400 to 500 RU HC2C1 or LC2C1 at a flow rate of 30 µl/min, followed by injection of different concentrations of the antigens at a flow rate of 30 µl/min. All kinetic data of the interaction were evaluated using BlAcore T200 evaluation software. The affinities and kinetic constants for are listed in Table 2.

**Table 2**

| Construct | human IL-17A | | | cynomolgus IL-17A | | | human TNF | | | cynomolgus TNF | | |
|---|---|---|---|---|---|---|---|---|---|---|---|---|
| | kₒₙ (1/Ms) | k_{off} (1/s) | K_{D} (pM) | kₒₙ (1/Ms) | k_{off} (1/s) | K_{D} (pM) | kₒₙ (1/Ms) | k_{off} (1/s) | K_{D} (pM) | kₒₙ (1/Ms) | k_{off} (1/s) | K_{D} (pM) |
| HC2C1 | 2.6 x 10⁵ | 6.6 x 10⁻⁵ | 252 | 3.6 x 10⁵ | 1.6 x 10⁻⁴ | 448 | 3.9 x 10⁵ | 2.8 x 10⁻⁵ | 70 | 8.2 x 10⁵ | 8.1 x 10⁻⁵ | 100 |
| LC2C1 | 2.3 x 10⁵ | 6.1 x 10⁻⁵ | 268 | 1.2 x 10⁵ | 4.6 x 10⁻⁵ | 398 | 4.0 x 10⁵ | 6.7 x 10⁻⁵ | 165 | 6.3 x 10⁵ | 8.8 x 10⁻⁵ | 140 |

### Example 4:

### In vitro inhibition of IL-17A and TNF

IL-17A and TNF induce the production of IL-6 in fibroblasts in a dose-dependent manner. The inhibitory activities of the indicated constructs were tested by stimulating human dermal fibroblasts with recombinant IL-17A and TNF in the absence or presence of various concentrations of HC2C1 and LC2C1. Cell culture supernatants were taken after 24 h of stimulation and assayed for IL-6 with ELISA.

### Method

100 µl of a cell suspension containing about 3900 Normal Human Dermal Fibroblasts (PromoCell, NHDF-c, C12300) were distributed per well (96 well plate, Nunc or TPP) and cultured for 24 hours at 37°C and 5% CO₂ (medium: Fibroblast Growth Medium C-23010, PromoCell). The supernatant was aspirated and after mixing different concentrations of HC2C1 or LC2C1 with medium containing recombinant IL-17A (produced in-house in EBNA cells) at a final concentration of 1 ng/ml and recombinant TNF (Thermo Fisher) at a final concentration of 50 pg/ml, 100 µl of the corresponding solution was added per well. As negative control, medium without IL-17A and TNF was added to the cells. Additionally IL-17A only, TNF only or the combination of the 2 cytokines were added to the cells as controls. After 24 hours incubation at 37°C the supernatant was removed and the IL-6 concentration was determined by ELISA according to the manufacturer's instructions (IL-6 ELISA kit, R&D Systems).

### Results

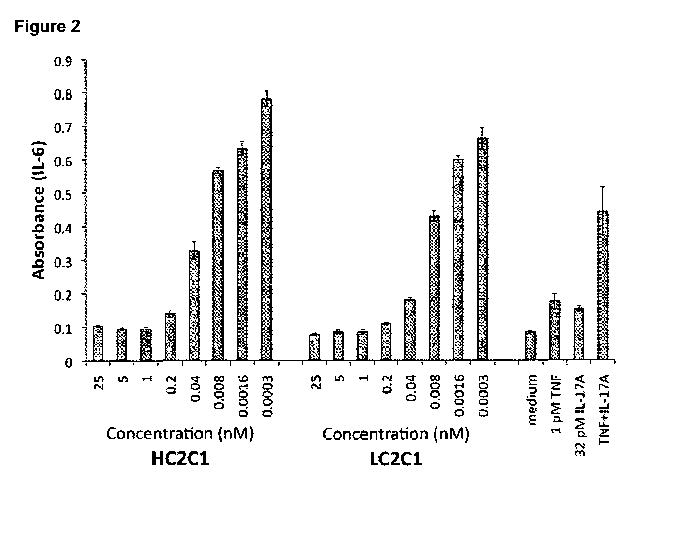

As shown in Figure 2 the inhibitory activities of HC2C1 and LC2C1 are comparable and obtained apparent IC₅₀ values are shown in Table 3.

**Table 3: Apparent IC₅₀ values for the inhibition of IL-17A and TNF**

| Construct | SEQ ID NOs (heavy chain, light chain) | Apparent IC₅₀ (pM) |
|---|---|---|
| HC2C1 | 125, 1 | 21 |
| LC2C1 | 119, 124 | 10 |

### Example 5:

### In vivo half-life

The *in vivo* half-life of the fusion proteins of the invention LC2C1 and HC2C1 were determined by measuring the concentrations by ELISA in mouse serum taken at different time points after a single i.v. injection.

### Methods

LC2C1 and HC2C1 were injected intravenously into 5 mice (C57BU6, Charles River) at a dose of 10mg/kg. After 10 minutes, 6, 24, 48, 72, 96, 120, 144 and 168 hours about 20 µl of blood was taken from the vena saphena with the capillary Microvette CB 300 (Sarstedt). Blood samples were centrifuged for 10 min at 9500 x g and the serum was stored at -20° until ELISA analysis was performed. Using dilution series with known concentrations of LC2C1 and HC2C1, the LC2C1 and HC2C1 concentration in serum was determined by ELISA using both TNF and IL-17A as capturing agents: 50 µl of biotinylated IL-17A (120 nM) (R&D Systems, biotinylated using EZ-link NHS-PGE4-biotin (Pierce) according to the manufacturer's instructions) or 50 µl of biotinylated TNF (10 nM) (Thermo Scientific, biotinylated using EZ-link NHS-PGE4-biotin (Pierce) according to the manufacturer's instructions) were added to streptavidin-coated wells (Reactibind, Pierce) and after blocking with 200 µl PBS, 4% milk (Rapilait, Migros, Switzerland), 50 µl of diluted serum samples (in PBS, 4% milk) was added. After incubation for 1 h and washing with PBS, bound antibody Fynomer fusion proteins were detected with protein A-HRP conjugate (Sigma). Peroxidase activity was detected by addition of QuantaRed enhanced chemifluorescent HRP substrate (Pierce). Fluorescence intensity was measured after 1 to 5 min at 544 nm (excitation) and 590 nm (emission). From the concentrations of LC2C1 and HC2C1 determined by ELISA in serum (n ≥5 per time point) at different time points after injection and the resulting slope k of the elimination phase (plotted in a semi-logarithmic scale), the half-lives (t_{1/2})of LC2C1 and HC2C1 were calculated using the formula t_{1/2} = ln2/-k.

### Results

The serum concentrations measured in the samples taken at different time points after injection are shown in Figure 4. The half-lives of the fusion proteins of the invention LC2C1 and HC2C1 and the final concentrations 168 hours after injection are given below in Table 4.

**Table 4**

| Format | t_{1/2} | Concentration (168 h) |
|---|---|---|
| HC2C1 (IL-17) | 58 h | 0.9 µg/ml |
| HC2C1 (TNF) | 41 h | 0.6 µg/ml |
| LC2C1 (IL-17) | 85 h | 3.1 µg/ml |
| HC2C1 (TNF) | 76 h | 3.6 µg/ml |

## Claims

1. A fusion protein comprising or consisting of the antibody light chain consisting of SEQ ID NO: 1 and a polypeptide binding to IL-17, wherein said polypeptide binding to IL-17 is located downstream of the C-terminus of said antibody light chain within said fusion protein, and wherein said polypeptide consists of the amino acid sequence of SEQ ID NO: 2, wherein
(a) one, two or three amino acids within amino acids positions 10 to 19 of SEQ ID NO: 2 is/are substituted, deleted or added,
(b) one, two or three amino acids within amino acids positions 29 to 36 of SEQ ID NO: 2 is/are substituted, deleted or added, and/or
(c) the amino acid position 48 is substituted or deleted,
provided that the polypeptide binding to IL-17 has at least 85% sequence identity to the amino acid sequence of SEQ ID NO: 2, wherein the determination of identity excludes amino acid positions 12 to 17, 31 to 34 and 48 of SEQ ID NO: 2.

2. The fusion protein according to claim 1, wherein said polypeptide consists of an amino acid sequence selected from the group consisting of SEQ ID NOs: 109, 3 to 108 and 110 to 118.

3. The fusion protein according to claim 1 or 2, wherein said polypeptide is fused to the C-terminus of the antibody light chain.

4. The fusion protein according to claims 1 or 2, wherein said polypeptide is connected to the C-terminus of the antibody light chain via a linker.

5. The fusion protein according to claim 5, wherein the linker is a peptide consisting of a sequence selected from the group consisting of SEQ ID NOs: 120 to 122.

6. A bispecific construct comprising or consisting of at least one copy, preferably two copies of the fusion protein of any one of claims 1 to 5 and at least one copy, preferably two copies of the antibody heavy chain of SEQ ID NO: 119.

7. A pharmaceutical composition comprising the fusion protein of any one of claims 1 to 5, or the bispecific construct of claim 6.

8. The pharmaceutical composition of claim 7 for use in a method of treating or preventing an inflammatory, autoimmune and/or bone loss-related disease.

9. The pharmaceutical composition for use according to claim 8, wherein said disease is selected from the group consisting of arthritis, preferably rheumatoid arthritis, arthritis chronica progrediente, reactive arthritis, psoriatic arthritis, enterophathic arthritis and arthritis deformans, rheumatic diseases, spondyloarthropathies, ankylosing spondylitis, Reiter syndrome, hypersensitivity (including both airways hypersensitivity and dermal hypersensitivity), allergies, systemic lupus erythematosus, inflammatory muscle disorders, polychondritis, sclerodoma, Wegener granulomatosis, dermatomyositis, Steven-Johnson syndrome, chronic active hepatitis, myasthenia gravis, psoriasis, idiopathic sprue, autoimmune inflammatory bowel disease, ulcerative colitis, Crohn's disease, Irritable Bowel Syndrome, endocrine ophthalmopathy, Graves disease, sarcoidosis, multiple sclerosis, primary biliary cirrhosis, juvenile diabetes (diabetes mellitus type I), autoimmune haematological disorders, hemolytic anaemia, aplastic anaemia, pure red cell anaemia, idiopathic thrombocytopenia, uveitis (anterior and posterior), keratoconjunctivitis sicca, vernal keratoconjunctivitis, interstitial lung fibrosis, glomerulonephritis (with and without nephrotic syndrome), idiopathic nephrotic syndrome or minimal change nephropathy, tumors, inflammatory disease of skin, cornea inflammation, myositis, loosening of bone implants, metabolic disorders, atherosclerosis, diabetes, and dislipidemia, bone loss, osteoarthritis, osteoporosis, periodontal disease of obstructive or inflammatory airways diseases asthma, bronchitis, pneumoconiosis, pulmonary emphysema, acute and hyperacute inflammatory reactions, diseases involving IL-17A-mediated TNF-alpha, acute infections, septic shock, endotoxic shock, adult respiratory distress syndrome, meningitis, pneumonia, severe burns, cachexia wasting syndrome, stroke, herpetic stromal keratitis and dry eye disease.

10. A nucleic acid molecule encoding the fusion protein of any one of claims 1 to 5, or at least one nucleic acid molecule encoding the bispecific construct of claim 6.

11. At least one vector comprising the nucleic acid molecule(s) of claim 10.

12. An isolated cell comprising the nucleic acid molecule(s) of claim 10 and/or the vector(s) of claim 11.

13. A method of producing the fusion protein of any one of claims 1 to 5, or the bispecific construct of claim 6 comprising
(a) culturing the isolated cell of claim 12, and
(b) isolating the produced fusion protein or bispecific construct.
